(19) Europäisches Patentamt
European Patent Office
Office européen des brevets

(11) Numéro de publication: **0 070 779 A2**

# (12) DEMANDE DE BREVET EUROPEEN

(21) Numéro de dépôt: **82401339.5**

(22) Date de dépôt: **19.07.82**

(51) Int. Cl.³: **C 07 D 233/22**, C 07 D 239/06, C 07 D 243/04, A 61 K 31/395

(30) Priorité: **17.07.81 FR 8114011**

(43) Date de publication de la demande: **26.01.83 Bulletin 83/4**

(84) Etats contractants désignés: **AT BE CH DE FR GB IT LI LU NL SE**

(71) Demandeur: **CARPIBEM (CENTRE D'ACTIVITE ET DE RECHERCHE PHARMACEUTIQUE INDUSTRIELLE BIOLOGIQUE ET MEDICALE) Société Anonyme dite:, 128, rue Danton, F-92500 Rueil-Malmaison (FR)**

(72) Inventeur: **Teulon, Jean-Marie, 12, Résidence du Bel-Ebat 56, avenue de Verdun, F-78170 La Celle Saint Cloud (FR)**

(74) Mandataire: **Richebourg, Michel François et al, Cabinet Beau de Loménie 55, rue d'Amsterdam, F-75008 Paris (FR)**

(54) **Nouveaux phénols di-ortho substitués dont une des substitutions est un hétérocycle, leur procédé de préparation, médicaments les contenant, notamment anti-hypertenseurs et nouveaux intermédiaires de synthèse.**

(57) L'invention concerne de nouveaux composés de formule:

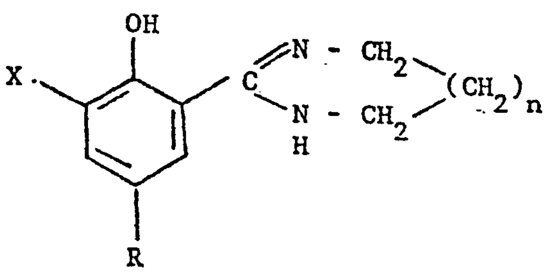

(I)

Anti-hypertenseurs.

EP 0 070 779 A2

0070779

Nouveaux phénols di-orthosubstitués dont une des substitutions est un hétérocycle, leur procédé de préparation, médicaments les contenant notamment anti-hypertenseurs et nouveaux intermédiaires de synthèse.

La présente invention concerne les phénols aminés de formule (I) et leurs sels d'addition d'acides. Elle concerne également le procédé de préparation desdits produits et leur application en thérapeuthique. Elle concerne en outre les composés intermédiaires nouveaux permettant la synthèse desdits produits.

Les nouveaux composés selon l'invention sont choisis parmi l'ensemble constitué par :

a) les composés de formule générale (I) :

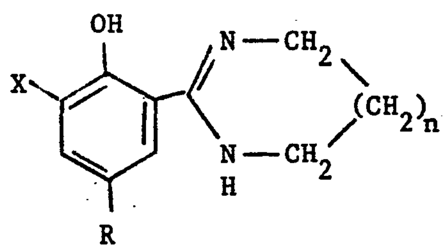

(I)

dans laquelle :

X représente un atome d'halogène, un groupement nitro, méthoxy, thiométhyl, SO $CH_3$, $SO_2$ $CH_3$ , acétamido ou allyle.

R représente un atome d'hydrogène, un radical alkyle notamment le radical terbutyle ou isobutyle, un radical cycloalkyle notamment le radical cyclohexyle, un radical alkylcycloalkyle notamment le radical méthylcyclopentyle, un radical alcoxy, un radical thioalkyle ou un atome d'halogène.

n est un nombre entier égal à 0, 1 ou 2

b) leurs sels d'addition d'acides.

Les composés de formule (I) selon l'invention peuvent être synthétisés:

1 - Soit par action d'un halogène ou d'un chlorure d'halogène sur un composé de formule (II) dans un solvant tel que par exemple l'acide acétique, à température ambiante, soit par action d'un N-halogénosuccinimide sur le composé de formule (II) dans un solvant tel que le diméthyl formamide, à température

ambiante, soit par action du chlorure de sulfuryle dans un solvant tel que par exemple l'acide acétique, à température ambiante, soit par nitration à l'aide de l'acide nitrique dans l'acide acétique, à température ambiante.

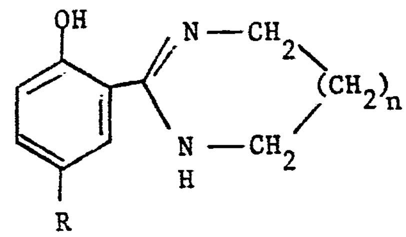

(II)

Dans la formule (II) R et n sont définis comme ci-dessus. D'une façon générale, les composés de formule (II) peuvent être obtenus par réaction d'un composé de formule (III) avec une diamine de formule :

$$H_2N - CH_2 - (CH_2)_n - CH_2 - NH_2$$

où n est défini comme ci-dessus, à une température comprise entre 160°C et 250°C, en tube scellé, si nécessaire.

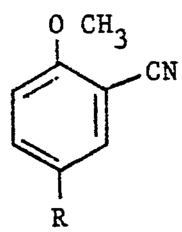

(III)

Dans la formule (III), R est défini comme ci-dessus.

Les nitriles de formule (III) peuvent être obtenus à partir d'aldéhydes de formule (IV) par des méthodes connues en soi telles que par exemple l'action de l'acide formique, du chlorhydrate d'hydroxylamine et du formiate de sodium au reflux.

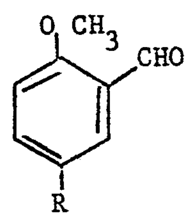

(IV)

Dans la formule (IV), R est défini comme ci-dessus.

Les aldéhydes de formule (IV) peuvent être préparés par des méthodes classiques telles que la réaction de Sommelet :

action de l'hexaméthylène tétramine sur les dérivés chlorométhylés de formule (V) ou encore l'action du sel de sodium du nitropropane sur les mêmes dérivés de formule (V) :

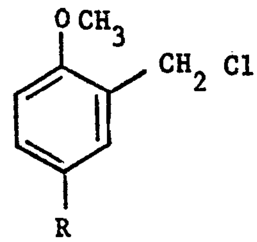

(V)

Dans la formule (V), R est défini comme ci-dessus.

Les dérivés chlorométhylés de formule (V) sont préparés par une réaction classique de chlorométhylation de Blanc : action de formol en présence d'acide chlorhydrique sur les dérivés de formule (VI) :

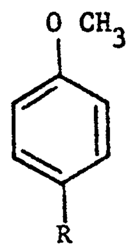

(VI)

Dans la formule (VI), R est défini comme ci-dessus.

2 - Soit par action d'une diamine de formule :

$$H_2N - CH_2 - (CH_2)_n - CH_2 - NH_2$$

où n est défini comme ci-dessus, à une température comprise entre 160°C et 250°C en tube scellé si nécessaire sur un phénol ortho nitrile de formule (VII) :

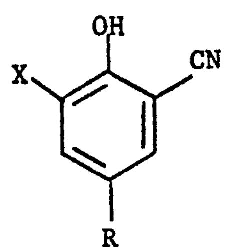

(VII)

dans la formule (VII), R et X sont définis comme ci-dessus.

Les nitriles de formule (VII) peuvent être obtenus à partir d'aldéhydes de formule (VIII) par des méthodes connues en soi telles que par exemple l'action de l'acide formique, du chlorhydrate d'hydroxylamine et du formiate de sodium au reflux

ou par chauffage dans l'anhydride acétique de l'oxime obtenue à partir de l'aldéhyde de formule (VIII)

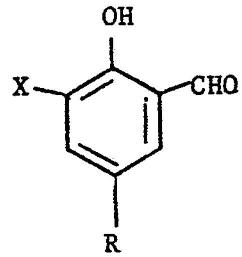

(VIII)

dans la formule (VIII) R et X sont définis comme ci-dessus.

Les aldéhydes de formule (VIII) peuvent être obtenus soit par halogénation des aldéhydes de formule (VIII) dans lesquels X = H soit dans le cas où X = allyle par la réaction classique de réarrangements de Claisen sur un aldéhyde de formule (VIII) où X = H ou encore peuvent être obtenus par une réaction classique d'introduction de la fonction aldéhyde en ortho d'un phénol tel que par exemple la réaction de Duff sur un phénol de formule (IX).

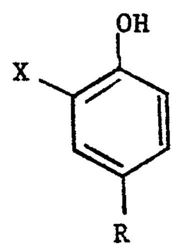

(IX)

dans la formule (IX), R et X sont définis comme ci-dessus.

Les sels d'addition d'acides des composés de formule (I) peuvent être obtenus par réaction avec un acide minéral ou organique, selon une méthode connue en soi. Parmi les acides utilisables à cet effet, on peut notamment citer les acides chlorhydrique, bromhydrique, sulfurique, phosphorique, oxalique, succinique, méthane sulfonique, cyclohexylsulfamique, formique, aspartique, glutamique, N-acétyl aspartique, N-acétyl glutamique, ascorbique, maléïque, malique, fumarique, lactique, benzoïque, cinnamique, p-toluène

sulfonique.

Selon l'invention, on propose des compositions thérapeutiques utiles notamment pour le traitement de l'hypertension, caractérisées en ce qu'elles renferment, en association avec un excipient physiologiquement acceptable, au moins un composé de formule (I) ou l'un de ses sels d'addition d'acides non toxiques.

L'invention concerne en outre les produits intermédiaires nouveaux de formule (II), (III) et (VII) notamment.

D'autres caractéristiques et avantages de l'invention seront mieux compris à la lecture qui va suivre de quelques exemples de préparation nullement limitatifs mais donnés à titre d'illustration.

Le tableau I, ci-après donne la formule développée de certains produits.

EXEMPLE I

p-Cyclohexyl o-chlorométhylanisole

formule (V) R = 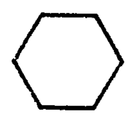

On porte à 60°C durant 26 h un mélange de 120 g de p-cyclohexylanisole, 17,1 g de formol(trioxyméthylène) et 190 ml d'acide chlorhydrique 36 %.

Le mélange réactionnel est ensuite refroidi et extrait à l'éther.

La phase éthérée est ensuite lavée à l'eau, à la soude 5% puis encore à l'eau et séchée sur sulfate de sodium. L'éther est évaporé et le résidu est soumis à distillation sous vide. On récupère ainsi 93,9 g de p-cyclohexyl o-chlorométhylanisole. E. = 130 - 145°C/1mm Hg.

EXEMPLE 2

2-Méthoxy 5-ter butylbenzaldéhyde

formule (IV) R = 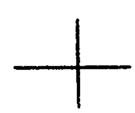 (t-Bu)

A une solution de méthylate de sodium préparée à partir de 10,8 g de sodium dans 470 ml de méthanol, on ajoute goutte à goutte 43,2 g de nitro-2 propane. On laisse le mélange sous agitation durant 30 min puis on additionne goutte à goutte, en maintenant la température inférieure à 45°C, 100 g de p-ter butyl o-chlorométhylanisole. Après la fin de l'addition, le mélange est agité durant 3 h en maintenant la température entre 40 et 45°C.

Le mélange réactionnel est ensuite concentré sous vide, repris à l'eau et extrait à l'éther. La phase éthérée est soigneusement lavée à l'eau, à la soude 10% et encore à l'eau, puis séchée sur sulfate de sodium.

L'éther est évaporé après filtration et le résidu obtenu est soumis à distillation sous vide. On récupère ainsi 68,2 g de 2-méthoxy 5-ter butylbenzaldéhyde. E. = 156-164°C/15 mm Hg.

EXEMPLE 3

2-Méthoxy 5-isobutylbenzaldéhyde

formule (IV) R = 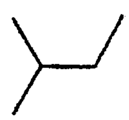 (i-Bu)

On porte au reflux durant 3 h le mélange de 117,3 g de p-isobutyl o-chlorométhylanisole, 47 ml d'eau distillée et 77 g d'hexaméthylènetétramine.

Le mélange réactionnel est ensuite soumis à l'entraînement à la vapeur d'eau.

La phase entraînée est alors extraite à l'éther qu'on lave soigneusement à l'eau, à la soude 5% et encore à l'eau, puis qu'on sèche sur sulfate de sodium.

L'éther est évaporé après filtration et le résidu obtenu est soumis à distillation sous vide. On récupère ainsi 56 g de 2-méthoxy 5-isobutylbenzaldéhyde. E. = 161-171°C/18 mm Hg.

EXEMPLE 4

2-Méthoxy 5-cyclohexylbenzaldéhyde

formule (IV) R = 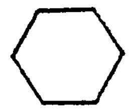

Selon le mode opératoire décrit à l'exemple 2, mais à partir de 93,9 g de p-cyclohexyl o-chlorométhylanisole préparé à l'exemple 1 et 36,2 g de nitro-2 propane sodé par une solution de méthylate de sodium préparée à partir de 9,1 g de sodium dans 400 ml de méthanol, on obtient après distillation sous vide 70 g de 2-méthoxy 5-cyclohexylbenzaldéhyde. E. = 140-150°C/0,5 mm Hg.

EXEMPLE 5

2-Méthoxy 5-ter butylbenzonitrile

formule (III) R = 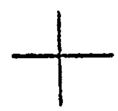 (t-Bu)

On porte au reflux durant 1 h un mélange de 68,2 g de 2-méthoxy 5-ter butylbenzaldéhyde préparé à l'exemple 2, 53,5 ml d'acide formique 98%, 25,4 g de chlorhydrate d'hydroxylamine et 25,4 g de formiate de sodium.

Le mélange réactionnel est ensuite refroidi, repris par un mélange d'eau et de glace et extrait à l'éther. La phase éthérée est lavée à l'eau, avec une solution aqueuse de bicarbonate, puis encore à l'eau et enfin séchée sur sulfate de sodium. Après filtration et évaporation de l'éther, le résidu obtenu est soumis à distillation sous vide. On récupère ainsi 48 g de 2-méthoxy 5-ter - butylbenzonitrile. E. = 120-130°C/0,5 mm Hg.

EXEMPLE 6

2-Méthoxy 5-isobutylbenzonitrile

formule (III) R = 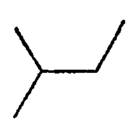 (iso-Bu)

Selon le mode opératoire décrit à l'exemple 5, mais en utilisant 56 g de 2-méthoxy 5-isobutylbenzaldéhyde préparé à l'exemple 3, 44 ml d'acide formique 98%, 20,8 g de chlorhydrate d'hydroxylamine et 20,8 g de formiate de sodium, on obtient après distillation sous vide 47,2 g de 2-méthoxy 5-isobutylbenzonitrile. E. = 120-132°C/0,6 mm Hg.

EXEMPLE 7

2-Méthoxy 5-cyclohexylbenzonitrile

formule (III) R = 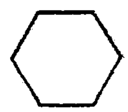

Selon le mode opératoire décrit à l'exemple 5, mais en utilisant 69,9 g de 2-méthoxy 5-cyclohexylbenzaldéhyde préparé à l'exemple 4, 48 ml d'acide formique 98%, 22,9 g de chlorhydrate d'hydroxylamine et 22,9 g de formiate de sodium, on obtient après distillation sous vide 49 g de 2-méthoxy 5-cyclohexylbenzonitrile. E. = 160-170°C/0,9 mm Hg.

EXEMPLE 8

2-(2'-Imidazolinyl) 4-ter butylphénol

formule (II) n = 0 R = 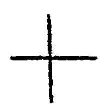 (t-Bu)

On porte à 165°C en tube scellé, 7 h, un mélange de 48 g de 2-méthoxy 5-ter butylbenzonitrile préparé à l'exemple 5 et 116 ml d'éthylènediamine.

Le mélange réactionnel est ensuite refroidi et les cristaux formés sont essorés et soigneusement lavés à l'isopropanol puis à l'éther. On récupère ainsi 35,2 g de cristaux. Par concentration des eaux mères, on obtient encore 8,5 g de cristaux de même point de fusion, soit au total 43,5 g de 2-(2'-imidazolinyl) 4-ter - butylphénol sous forme de cristaux de point de fusion 188-190°C.

EXEMPLE 9

2-(2'-Imidazolinyl) 4-isobutylphénol

formule (II) n = 0 R = 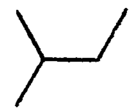 (iso-Bu)

Selon le mode opératoire de l'exemple 8, mais en utilisant 85 g de 2-méthoxy 5-isobutylbenzonitrile préparé à l'exemple 6 et 206 ml d'éthylènediamine, on obtient 72,3 g de 2-(2'-imidazolinyl) 4-isobutylphénol sous forme de cristaux de point de fusion 185-187°C.

EXEMPLE 10

2-(2'-Imidazolinyl) 4-cyclohexylphénol

formule (II) n = 0 R = 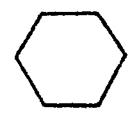

Selon le mode opératoire de l'exemple 8, mais en utilisant 49 g de 2-méthoxy 5-cyclohexylbenzonitrile préparé à l'exemple 7 et 118 ml d'éthylènediamine, on obtient 47,1 g de 2-(2'-imidazolinyl) 4-cyclohexylphénol sous forme de cristaux de point de fusion 194-196°C.

EXEMPLE 11

Bromhydrate de 2-(2'-imidazolinyl) 4-ter butyl 6-bromophénol

formule (I) n = 0 R = 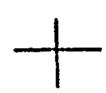 X = Br

(t-Bu)

A une solution de 13 g de 2-(2'-imidazolinyl) 4-ter - butylphénol préparé à l'exemple 8 dans 100 ml d'acide acétique glacial, on ajoute goutte à goutte, sous bonne agitation, une solution de 3,1 ml de brome dans 35 ml d'acide acétique glacial. Après la fin de l'addition, on continue l'agitation. Au bout de 1 h, on constate

que le produit commence à cristalliser. On continue l'agitation encore 4 h, puis les cristaux formés sont essorés, lavés avec un peu d'acétonitrile puis à l'éther. Le composé obtenu, 19 g, est recristallisé dans 70 ml d'isopropanol. On obtient ainsi 15,4 g de bromhydrate de 2-(2'-imidazolinyl) 4-ter butyl 6-bromophénol sous forme de cristaux blancs de point de fusion 226-227°C.

EXEMPLE 12

Chlorhydrate de 2-(2'-imidazolinyl) 4-ter butyl 6-iodophénol

formule (I) n = 0 R = 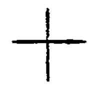 X = I

(t-Bu)

A une solution de 15 g de 2-(2'-imidazolinyl) 4-ter - butylphénol préparé à l'exemple 8 dans 120 ml d'acide acétique glacial, on ajoute par petites fractions 11,3 g de chlorure d'iode sous bonne agitation. Après la fin de l'addition, l'agitation est continuée durant 5 h puis le mélange réactionnel est laissé au repos durant une nuit. On ajoute ensuite au mélange 200 ml d'éther et les cristaux formés sont essorés puis lavés à l'éther. Les 12,3 g ainsi obtenus sont recristallisés dans 100 ml d'isopropanol. On récupère ainsi 6,5 g de chlorhydrate de 2-(2'-imidazolinyl) 4-ter butyl 6-iodophénol sous forme de cristaux blancs de point de fusion 196-200°C.

EXEMPLE 13

Bromhydrate de 2-(2'-imidazolinyl) 4-isobutyl 6-bromophénol

formule (I) n = 0 R = 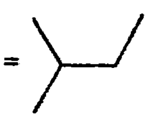 X = Br

(iso-Bu)

Selon le mode opératoire de l'exemple 11, mais en utilisant 12 g de 2-(2'-imidazolinyl) 4-isobutylphénol préparé à l'exemple 9 et 2,8 ml de brome, on obtient après recristallisation dans l'isopropanol 13,3 g de bromhydrate de 2-(2'-imidazolinyl) 4-isobutyl 6-bromophénol sous forme de cristaux blancs de point de fusion 235-236°C.

EXEMPLE 14

Chlorhydrate de 2-(2'-imidazolinyl) 4-isobutyl 6-iodophénol monohydraté

formule (I) n = 0 R = 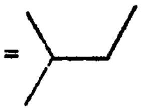 X = I

(iso-Bu)

Selon le mode opératoire de l'exemple 12, mais en utilisant 10 g de 2-(2'-imidazolinyl) 4-isobutylphénol préparé à l'exemple 9 et 7,5 g de chlorure d'iode, on obtient après recristallisation dans le chloroforme 8,2 g de chlorhydrate de 2-(2'-imidazolinyl) 4-isobutyl 6-iodophénol monohydrate sous forme de cristaux blancs de point de fusion 170-172°C.

EXEMPLE 15

Bromhydrate de 2-(2'-imidazolinyl) 4-cyclohexyl 6-bromophénol

formule (I) n = 0 R = 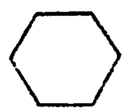 X = Br

Selon le mode opératoire de l'exemple 11, mais en utilisant 12 g de 2-(2'-imidazolinyl) 4-cyclohexylphénol préparé à l'exemple 10 et 2,5 ml de brome, on obtient 18 g de bromhydrate de 2-(2'-imidazolinyl) 4-cyclohexyl 6-bromophénol sous forme de cristaux blancs de point de fusion 218-220°C.

EXEMPLE 16

Chlorhydrate de 2-(2'-imidazolinyl) 4-cyclohexyl 6-iodophénol

formule (I) n = 0 R = 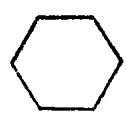 X = I

Selon le mode opératoire de l'exemple 12, mais en utilisant 15 g de 2-(2'-imidazolinyl) 4-cyclohexylphénol préparé à l'exemple 10 et 10 g de chlorure d'iode, on obtient 16,4 g de chlorhydrate de 2-(2'-imidazolinyl) 4-cyclohexyl 6-iodophénol sous forme de cristaux blancs de point de fusion 193-195°C.

EXEMPLE 17

2-(2'-Tétrahydro-3',4',5',6' pyrimidinyl) 4-isobutylphénol

formule (II) n = 1 R = 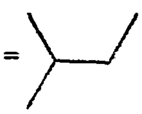 (iso-Bu)

On porte à 165°C en tube scellé durant 8 h un mélange de 15 g de 2-méthoxy 5-isobutylbenzonitrile préparé à l'exemple 6 et 125 ml de 1,3-diaminopropane.

Le mélange réactionnel est ensuite refroidi et les cristaux formés sont essorés et soigneusement lavés à l'isopropanol puis à l'éther.

On récupère ainsi 14,1 g de 2-(2'-tétrahydro-3',4',5',6' pyrimidinyl) 4-isobutylphénol sous forme de cristaux de point de fusion 229-232°C.

EXEMPLE 18

Bromhydrate de 2-(2'-tétrahydro-3',4',5',6' pyrimidinyl) 4-isobutyl 6-bromophénol

formule (I) n = 1 R = 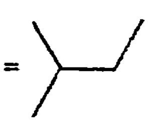 X = Br

(iso-Bu)

A une solution de 7 g de 2-(2'-tétrahydro-3',4',5',6' pyrimidinyl) 4-isobutylphénol préparé à l'exemple 17 dans 50 ml d'acide acétique glacial, on ajoute goutte à goutte, sous bonne agitation, une solution de 1,6 ml de brome dans 15 ml d'acide acétique glacial. Après la fin de l'addition, on continue l'agitation.

Au bout de 1 h, on observe que le produit commence à cristalliser. On continue l'agitation encore 4 h, puis on laisse au repos une nuit. Les cristaux formés sont ensuite essorés puis soigneusement lavés avec un peu d'acide acétique puis de l'éther. On récupère ainsi 10,2 g de bromhydrate de 2-(2'-tétrahydro-3',4',5',6' pyrimidinyl) 4-isobutyl 6-bromophénol sous forme de cristaux blancs de point de fusion 196-199°C.

## EXEMPLE 19

Chlorhydrate de 2-(2'-tétrahydro-3',4',5',6' pyrimidinyl) 4-isobutyl 6-iodophénol

formule (I) n = 1 R = 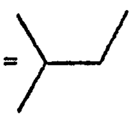 (iso-Bu) X = I

A une solution de 1,6 g de 2-(2'-tétrahydro-3',4',5',6' pyrimidinyl) 4-isobutylphénol préparé à l'exemple 17 dans 15 ml d'acide acétique glacial, on ajoute par petites fractions 1,15 g de chlorure d'iode sous bonne agitation. Après la fin de l'addition, l'agitation est continuée durant 5 h puis le mélange réactionnel est laissé au repos durant une nuit. On ajoute ensuite au mélange 30 ml d'éther et les cristaux formés sont essorés puis lavés à l'éther. Après recristallisation dans l'isopropanol, on récupère 1,1 g de chlorhydrate de 2-(2'-tétrahydro-3',4',5',6' pyrimidinyl) 4-isobutyl 6-iodophénol sous forme de cristaux blancs de point de fusion 190-192°C.

## EXEMPLE 20

2-(2'-Tétrahydro-3',4',5',6' pyrimidinyl) 4-ter butylphénol

formule (II) n = 1 R = 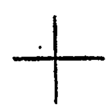 (t-Bu)

Selon le mode opératoire de l'exemple 17, mais en utilisant 20 g de 2-méthoxy 5-ter butylbenzonitrile préparé à l'exemple 5 et 165 ml de 1,3-diaminopropane, on obtient 18,6 g de 2-(2'-tétrahydro-3',4',5',6' pyrimidinyl) 4-ter butylphénol sous forme de cristaux de point de fusion 285-287°C.

EXEMPLE 21

Bromhydrate de 2-(2'-tétrahydro-3',4',5',6' pyrimidinyl) 4-ter butyl 6-bromophénol

formule (I) n = 1 R = 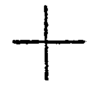 X = Br

(t-Bu)

Selon le mode opératoire de l'exemple 18, mais en utilisant 5 g de 2-(2'-tétrahydro-3',4',5',6' pyrimidinyl) 4-ter - butylphénol préparé à l'exemple 20 et 1,1 ml de brome, on obtient 7,6 g qui, recristallisés dans le mélange acétonitrile-isopropanol 7-3, donnent 4,7 g de bromhydrate de 2-(2'-tétrahydro-3',4',5',6' pyrimidinyl) 4-ter butyl 6-bromophénol sous forme de cristaux blancs de point de fusion 210-211°C.

EXEMPLE 22

Chlorhydrate de 2-(2'-tétrahydro-3',4',5',6' pyrimidinyl) 4-ter - butyl 6-iodophénol

formule (I) n = 1 R = 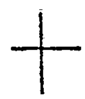 X = I

(t-Bu)

Selon le mode opératoire de l'exemple 19, mais en utilisant 6 g de 2-(2'-tétrahydro-3',4',5',6' pyrimidinyl) 4-ter - butylphénol préparé à l'exemple 20 et 4,2 g de chlorure d'iode, on obtient 8,5 g qui, recristallisés dans le mélange acétonitrile-isopropanol 7-3, donnent 5,9 g de chlorhydrate de 2-(2'-tétrahydro-3',4',5',6' pyrimidinyl) 4-ter butyl 6-iodophénol sous forme de cristaux blancs de point de fusion 207-209°C décomposition.

EXEMPLE 23

2-(2'-Tétrahydro-3',4',5',6' pyrimidinyl) 4-cyclohexylphénol

formule (II) n = 1 R = 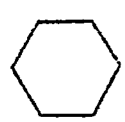

Selon le mode opératoire de l'exemple 17, mais en utilisant 30 g de 2-méthoxy 5-cyclohexylbenzonitrile préparé à l'exemple 7 et 72 ml de 1,3-diaminopropane, on obtient 20,5 g de 2-(2'-tétrahydro-3',4',5',6' pyrimidinyl) 4-cyclohexylphénol sous forme de cristaux de point de fusion 244-248°C.

EXEMPLE 24

Bromhydrate de 2-(2'-tétrahydro-3',4',5',6' pyrimidinyl) 4-cyclohexyl 6-bromophénol

formule (I) n = 1 R = 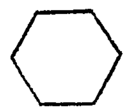 X = Br

Selon le mode opératoire de l'exemple 18, mais en utilisant 7,5 g de 2-(2'-tétrahydro-3',4',5',6' pyrimidinyl) 4-cyclohexylphénol préparé à l'exemple 23 et 1,5 ml de brome, on obtient 10,9 g de cristaux qui, recristallisés dans 60 ml d'isopropanol, donnent 8,9 g de bromhydrate de 2-(2'-tétrahydro-3',4',5',6' pyrimidinyl) 4-cyclohexyl 6-bromophénol sous forme de cristaux blancs de point de fusion 195-197°C.

EXEMPLE 25

Chlorhydrate de 2-(2'-tétrahydro-3',4',5',6' pyrimidinyl) 4-cyclohexyl 6-iodophénol

formule (I) n = 1 R = 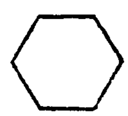 X = I

Selon le mode opératoire de l'exemple 19, mais en utilisant 7,5 g de 2-(2'-tétrahydro-3',4',5',6' pyrimidinyl) 4-cyclohexylphénol préparé à l'exemple 23 et 4,7 g de chlorure d'iode, on obtient 9,6 g de cristaux qui, recristallisés dans 100 ml d'isopropanol, conduisent à 6,9 g de chlorhydrate de 2-(2'-tétrahydro-3',4',

5',6' pyrimidinyl) 4-cyclohexyl 6-iodophénol sous forme de cristaux blancs de point de fusion 203-205°C décomposition.

EXEMPLE 26

2-(2'-tétrahydro-4',5',6',7' [1H diazépine(1,3)])4-ter butylphénol

formule (II) n = 2 R = 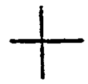 (t-Bu)

On porte à 160°C dans un ballon sous bonne agitation durant 8 h un mélange de 40 g de 2-méthoxy 5-ter butylbenzonitrile préparé à l'exemple 5 et de 96 ml de 1,4-diaminobutane.

Le mélange réactionnel est ensuite refroidi et les cristaux formés sont agités avec 50 ml d'isopropanol puis essorés et soigneusement lavés à l'isopropanol puis à l'éther. Après recristallisation dans le méthanol, on obtient 30,1 g de 2-(2'-tétrahydro-4',5',6',7' [1H diazépine(1,3)])4-ter butylphénol sous forme de cristaux de point de fusion 285-286°C.

EXEMPLE 27

Bromhydrate de 2-(2'-tétrahydro-4',5',6',7' [1H diazépine(1,3)]) 4-ter butyl 6-bromophénol

formule (I) n = 2 R = 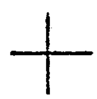 X = Br

(t-Bu)

A une solution de 7 g de 2-(2'-tétrahydro-4',5',6',7' [1H diazépine(1,3)])4-ter butylphénol préparé à l'exemple 26 dans 40 ml d'acide acétique glacial, on ajoute goutte à goutte, sous bonne agitation, une solution de 1,5 ml de brome dans 5 ml d'acide acétique glacial. Après la fin de l'addition, on continue l'agitation. Au bout de 1 h, on constate que le produit commence à cristalliser. On continue l'agitation encore 4 h et on laisse au repos une nuit. Les cristaux formés sont ensuite essorés puis soigneusement lavés à l'éther. On récupère ainsi 6,5 g de 2-(2'-tétrahydro-4',5',6',7' [1H diazépine(1,3)])4-ter butyl 6-bromophénol sous forme de cristaux blancs de point de fusion 190-193°C.

Par addition de l'éther de lavage avec les eaux mères, on peut encore récupérer 4 g du même composé de même point de fusion, soit au total 10,5 g.

La recristallisation de 10 g dans 80 ml d'acétonitrile conduit à 7,2 g de cristaux blancs de point de fusion 192-193°C.

EXEMPLE 28

Chlorhydrate de 2-(2'-tétrahydro-4',5',6',7' [1H diazépine(1,3)]) 4-ter butyl 6-iodophénol

formule (I) n = 2 R = 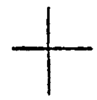 X = I

(t-Bu)

A une solution de 7 g de 2-(2'-tétrahydro-4',5',6',7' [1H diazépine(1,3)] 4-ter butylphénol préparé à l'exemple 26 dans 40 ml d'acide acétique glacial, on ajoute par petites fractions 4,6 g de chlorure d'iode sous bonne agitation. Après la fin de l'addition, l'agitation est continuée pendant 5 h puis le mélange réactionnel est laissé au repos durant une nuit. On ajoute ensuite au mélange 40 ml d'éther et les cristaux formés sont essorés puis soigneusement lavés à l'éther, avec un peu d'isopropanol puis encore à l'éther. On récupère ainsi 9,5 g de chlorhydrate de 2-(2'-tétrahydro-4',5',6',7' [1H diazépine(1,3)]) 4-ter butyl 6-iodophénol sous forme de cristaux blancs de point de fusion 179-182°C.

La recristallisation de 9 g dans 100 ml d'acétonitrile conduit à 5,5 g de cristaux blancs de point de fusion 182-183°C décomposition.

EXEMPLE 29

Chlorhydrate de 2-(2'-imidazolinyl) 4-isobutyl 6-chloro phénol

Formule (I) n = o R = 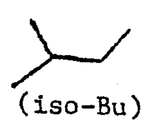 X = Cl

(iso-Bu)

Dans une solution de 10 g de 2-(2'-imidazolinyl)4-isobutyl phénol préparé à l'exemple 9, on fait barboter sous agitation du chlore gazeux jusqu'à absorption de 4 g. La réaction est exothermique. Une fois l'absorption terminée, le mélange réactionnel est

agité 2 heures puis abandonné une nuit. En ajoutant de l'éther, un précipité se forme qui cristallise dans l'acétone pour donner 8,1 g d'un mélange du phénol de départ et du produit attendu. Ce mélange est repris dans un minimum d'eau puis alcalinisé par du bicarbonate de sodium. L'eau est ensuite décantée et le résidu gommeux cristallise dans l'éther pour donner 6,7 g de base. On filtre cette base sur une colonne de 150 g de Silicagel dans le chlorure de méthylène. Les 6,7 g de base précédemment obtenus sont déposés sur la colonne puis élués par un mélange de chlorure de méthylène et de méthanol (90-10). On obtient deux fractions. La première fraction est concentrée sous vide puis lavée à l'éther pour donner 3,5 g de 2-(2'-imidazolinyl) 4-isobutyl 6-chloro phénol sous forme de base fondant à 250°C.

Chlorhydrate :

La base précédemment obtenue est mise en suspension dans l'acétone et acidifiée par une solution saturée d'éther chlorhydrique. Le mélange réactionnel est agité pendant 2 heures et le précipité est alors essoré. Après lavage à l'acétone puis à l'éther, on obtient 3,7 g de chlorhydrate de 2-(2'-imidazolinyl) 4-isobutyl 6-chloro phénol sous forme de cristaux blancs fondant à 237°C.

EXEMPLE 30

2-hydroxy 3-chloro 5-terbutyl benzonitrile

formule VII X = Cl R = terbutyle.

On porte au reflux durant 1 h un mélange de 31,3 g de 2-hydroxy 3-chloro 5-terbutyl benzaldéhyde, 31,3 ml de pyridine et 31,3 g de chlorhydrate d'hydroxylamine dans 125 ml d'éthanol. La solution est ensuite concentrée sous vide, additionnée d'eau et extraite à éther qu'on sèche sur sulfate de sodium, filtre et qu'on évapore. Le résidu obtenu : 38,8 g d'oxime est repris dans 210 ml d'anhydride acétique et chauffé au reflux durant 3 h 30. Après concentration sous vide, on additionne une solution aqueuse de 22,2 g de potasse dans 450 ml d'eau et on chauffe à 80-90°C jusqu'à passage en solution : 15 minutes. La solution aqueuse refroidie est lavée à l'éther puis acidifiée par une solution aqueuse d'acide chlorhydrique normale et les produits organiques

sont extraits de l'éther. Après avoir séché la phase éthérée sur sulfate de sodium, filtré et évaporé on récupère 29,8 g de 2-hydroxy 3-chloro 5-tertbutyl benzonitrile sous forme de cristaux de point de fusion 80-82°C.

EXEMPLE 31

2-(2'-imidazolinyl) 4-terbutyl 6-chlorophénol

formule I  n = 0  R = terbutyle  X = Cl

On porte à 165°C en tube scellé, 8 h, un mélange de 2-hydroxy 3-chloro 5-terbutyl benzonitrile préparé à l'exemple 30 et 72 ml d'éthylène diamine.

Le mélange réactionnel est ensuite refroidi et les cristaux formés sont essorés et soigneusement lavés à l'isopropanol puis à l'éther et séchés.

On récupère ainsi 21,6 g de 2-(2'-imidazolinyl) 4-terbutyl 6-chlorophénol sous forme de cristaux de point de fusion 257-260°C. (La recristallisation dans un mélange isopropanol-éthanol 50-50 donne le même point de fusion).

Chlorhydrate

Les 21,6 g de base sont dissous à chaud dans le méthanol et additionnés d'éther chlorhydrique jusqu'à pH acide. La solution est ensuite concentrée sous vide et reprise à l'éther. Les cristaux formés sont essorés, lavés à l'éther, à l'acétone puis encore à l'éther et séchés. On récupère 23,2 g de chlorhydrate de 2-(2'-imidazolinyl) 4-terbutyl 6-chlorophénol sous forme de cristaux de point de fusion 236-242°C. Après recristallisation dans 220 ml du mélange acétonitrile-eau 92-8 on récupère 20,6 g fondant à 240-4°C.

EXEMPLE 32

2-hydroxy 3-fluoro 5-terbutyl benzaldéhyde

formule VIII  X = F  R = terbutyle

Selon la méthode de DUFF, on opère de la façon suivante :

On chauffe à 165°C un mélange de 490 g de glycérol et 114,2 g d'acide borique et on laisse distiller l'azéotrope glycérol-eau jusqu'à l'obtention d'une température de 165°C dans le ballon réactionnel. On laisse alors refroidir jusqu'à 150°C et

on ajoute un mélange intime de 81,6 g de 2-fluoro 4-terbutyl phénol et de 81,6 g d'hexaméthylène tétramine par petites fractions en maintenant la température du mélange réactionnel entre 150°C et 165°C ; après la fin de l'addition cette température est encore maintenue durant 20 minutes.

On refroidit ensuite à 115°C et on ajoute un mélange de 86 ml d'acide sulfurique dans 246 ml d'eau.

On opère ensuite un entraînement à la vapeur en maintenant la température du ballon réactionnel à 140°C.

On distille ainsi 4 litres d'eau qui sont extraits au dichlorométhane qu'on sèche sur sulfate de sodium, filtre et qu'on évapore. On obtient ainsi 28,3 g de 2-hydroxy 3-fluoro 5-terbutyl benzaldéhyde sous forme d'un liquide qu'on utilise ainsi pour la suite des opérations.

EXEMPLE 33

2-hydroxy 3-fluoro 5-terbutyl benzonitrile

formule VII X = F R = terbutyle

Selon le mode opératoire de l'exemple 30 mais en utilisant 28,3 g de 2-hydroxy 3-fluoro 5-terbutyl benzaldéhyde préparé à l'exemple 32, on obtient 25,2 g de 2-hydroxy 3-fluoro 5-terbutyl benzonitrile sous forme de cristaux de point de fusion 110-112°C.

EXEMPLE 34

Chlorhydrate de 2-(2'-imidazolinyl) 4-terbutyl 6-fluorophénol

formule I n = 0 R = terbutyle X = F

Selon le mode opératoire de l'exemple 31 mais en utilisant 18,5 g de 2-hydroxy 3-fluoro 5-terbutyl benzonitrile préparé à l'exemple 33 et 44 ml d'éthylène diamine on récupère 17,9 g de base. Cette base est dissoute dans le méthanol et additionnée d'éther chlorhydrique jusqu'à pH acide. La solution est ensuite concentrée sous vide, reprise à l'éther et les cristaux obtenus sont lavés à l'éther, puis séchés.

On obtient ainsi 18 g de cristaux qui sont recristallisés dans 50 ml d'isopropanol ce qui conduit à 15 g du chlorhydrate de 2-(2'-imidazolinyl) 4-terbutyl 6-fluoro phénol sous forme de cristaux de point de fusion 272-3°C.

EXEMPLE 35

2-(2'-imidazolinyl) 4-terbutyl 6-nitrophénol

formule I n = 0 R = terbutyle X = $NO_2$

On additionne à une solution de 16 g de 2-(2'-imidazolinyl) 4-terbutylphénol préparés à l'exemple 8 du ler texte dans 75 ml d'acide acétique 3,6 ml d'acide nitrique de densité 1,49.

Le mélange réactionnel est agité à température ambiante (20-25°C) durant 1 h 30 minutes puis détruit sur de la glace.

La solution est neutralisée au bicarbonate de sodium et les cristaux obtenus sont essorés, lavés à l'eau puis à l'éther et enfin à l'isopronanol. Après séchage les cristaux sont recristallisés dans le méthanol. On récupère ainsi 4,1 g de 2-(2'-imidazolinyl) 4-terbutyl 6-nitrophénol sous forme de cristaux de point de fusion 301-303°C.

EXEMPLE 36

2-hydroxy 3-thiométhyl 5-terbutyl benzaldéhyde

formule VIII X = S $CH_3$ R = terbutyle

Selon la méthode de DUFF, en utilisant le mode opératoire de l'exemple 32 mais à partir de 112 g de 2-thiométhyl 4-terbutyl phénol on récupère 41,7 g de 2-hydroxy 3-thiométhyl 5-terbutyl benzaldéhyde sous forme de cristaux de point de fusion < 50°C

EXEMPLE 37

2-hydroxy 3-thiométhyl 5-terbutyl benzonitrile

formule VII X = S $CH_3$ R = terbutyle

Selon le mode opératoire de l'exemple 30 mais en utilisant 11,7 g de 2-hydroxy 3-thiométhyl 5-terbutyl benzaldéhyde préparé à l'exemple 36, on récupère 8,9 g de 2-hydroxy 3-thiométhyl 5-terbutyl benzonitrile sous forme de cristaux de point de fusion 70-3°C.

EXEMPLE 38

2-(2'-imidazolinyl) 4-terbutyl 6-thiométhyl phénol

formule I n = 0 R = terbutyle X = S $CH_3$

Selon le mode opératoire de l'exemple 31 mais en utilisant 11 g de 2-hydroxy 3-thiométhyl 5-terbutyl benzonitrile préparé à l'exemple 37 et 30 ml d'éthylène diamine on récupère 9 g

de 2-(2'-imidazolinyl) 4-terbutyl 6-thiométhyl phénol sous forme de cristaux de point de fusion 227-231°C.

Chlorhydrate

Les 9 g de base sont dissous à chaud dans le méthanol et additionnés d'éther chlorhydrique jusqu'à pH acide. La solution est ensuite concentrée sous vide et reprise à l'éther et les cristaux formés sont essorés, lavés à l'éther et séchés. Par recristallisation dans 200 ml d'acétonitrile on récupère 8,4 g de chlorhydrate de 2-(2'-imidazolinyl) 4-terbutyl 6-thiométhyl phénol sous forme de cristaux de point de fusion 193-7°C.

EXEMPLE 39

2-(2'imidazolinyl) 4-terbutyl 6-méthyl sulfinyl phénol

formule I n = 0 R = terbutyle X = S $OCH_3$

A une solution de 6,6 g de 2-(2'-imidazolinyl) 4-terbutyl 6-thiométhyl phénol préparés à l'exemple 38 dans 200 ml de méthanol, on ajoute à 0°C par petites quantités 5,3 g d'acide métachloroperbenzoïque. Après la fin de l'addition on laisse revenir le mélange réactionnel à température ambiante puis on le maintien sous agitation durant 12 h. On ajoute ensuite un mélange d'eau et de glace et on extrait au chloroforme.

La phase chloroformique est lavée au carbonate de sodium puis à l'eau et enfin après avoir séché et filtré on évapore le chloroforme. Le résidu obtenu cristallise dans l'acétone. On essore les cristaux qu'on lave avec un peu d'acétone et qu'on sèche. On obtient ainsi 4,7 g de 2-(2'-imidazolinyl) 4-terbutyl 6-méthyl sulfinyl phénol sous forme de cristaux de point de fusion 242-6°C.

Maléate

Les 4,7 g de base sont dissous dans le minimum de méthanol et additionnés de 1,9 g d'acide maléïque dissous dans le minimum de méthanol. Par addition d'un peu d'éther le sel cristallise. On essore les cristaux qu'on lave à l'éther et qu'on sèche. On récupère ainsi 4,8 g de maléate de 2-(2'-imidazolinyl) 4-terbutyl 6-méthyl sulfinyl phénol sous forme de cristaux de point de fusion 138-141°C.

EXEMPLE 40

2-hydroxy 3-méthoxy 5-terbutyl benzaldéhyde

formule VIII X = O $CH_3$ R = terbutyle

Selon la méthode de DUFF en utilisant le mode opératoire de l'exemple 32, mais à partir de 16,5 g de 2-méthoxy 4-terbutyl phénol on récupère 5,9 g de 2-hydroxy 3-méthoxy 5-terbutyl benzaldéhyde sous forme de cristaux de point de fusion 63-65°C.

EXEMPLE 41

2-hydroxy 3-méthoxy 5-terbutyl benzonitrile

formule VII X = O $CH_3$ R = terbutyle

Selon le mode opératoire de l'exemple 30 mais en utilisant 22,6 g de 2-hydroxy 3-méthoxy 5-terbutyl benzaldéhyde préparé à l'exemple 40 on récupère 20,5 g de 2-hydroxy 3-méthoxy 5-terbutyl benzonitrile sous forme de cristaux de point de fusion 74 - 6° C.

EXEMPLE 42

2-(2'-imidazolinyl) 4-terbutyl 6-méthoxy phénol

formule I n = 0 R = terbutyle X = O $CH_3$

Selon le mode opératoire de l'exemple 31 mais en utilisant 20,5 g de 2-hydroxy 3-méthoxy 5-terbutyl benzonitrile préparé à l'exemple 41 et 50 ml d'éthylène diamine on récupère 19 g de 2-(2'-imidazolinyl) 4-terbutyl 6-méthoxy phénol sous forme de cristaux de point de fusion 243-6°C.

Chlorhydrate

10 g de base sont dissous dans le méthanol à chaud et additionnés d'éther chlorhydrique jusqu'à pH acide. La solution est ensuite concentrée sous vide et reprise à l'éther et les cristaux formés sont essorés, lavés à l'éther et séchés. Les 10,9 g ainsi récupérés sont recristallisés dans 100 ml d'acétonitrile conduisant à 9 g du chlorhydrate de 2-(2'-imidazolinyl) 4-terbutyl 6-méthoxy phénol sous forme de cristaux de point de fusion 190-4°C.

EXEMPLE 43

2-hydroxy 3-allyl 5-terbutyl benzaldéhyde

formule VIII X = allyle R = terbutyle

On chauffe au reflux durant 8 h une solution de 40 g de 2-hydroxy 5-terbutyl benzaldéhyde, 22,6 ml de bromure d'allyle dans 90 ml d'acétone anhydre en présence de 30,9 g de carbonate de potassium sec.

Après refroidissement le mélange réactionnel est additionné d'eau puis extrait à l'éther. La phase éthérée est lavée à la soude puis à l'eau et séchée. Après évaporation de l'éther, le résidu : 47,4 g est repris dans 180 ml de diphényl éther et porté à la température de 240°C durant 1 h 30.

Après refroidissement on ajoute de l'éther et on extrait avec une solution de soude. La phase basique est lavée à l'éther puis acidifiée avec une solution d'acide chlorhydrique normale. Le phénol est alors extrait à l'éther qu'on lave à l'eau, sèche sur sulfate de sodium, filtre et évapore. On obtient ainsi 27,2 g de 2-hydroxy 3-allyl 5-terbutyl benzaldéhyde sous forme d'une huile qu'on utilise pour la suite des opérations.

EXEMPLE 44

2-hydroxy 3-allyl 5-terbutyl benzonitrile

formule VII X = allyle R = terbutyle

Selon le mode opératoire de l'exemple 30 mais en utilisant 27,2 g de 2-hydroxy 3-allyl 5-terbutyl benzaldéhyde préparé à l'exemple 43 on récupère 19,3 g de 2-hydroxy 3-allyl 5-terbutyl benzonitrile sous forme de cristaux de point de fusion 72-4°C.

EXEMPLE 45

2-(2'-imidazolinyl) 4-terbutyl 6-allyl phénol

formule I n = 0 R = terbutyle X = allyle

Selon le mode opératoire de l'exemple 31 mais en utilisant 19,3 g de 2-hydroxy 3-allyl 5-terbutyl benzonitrile préparé à l'exemple 44 et 48 ml d'éthylène diamine on récupère 12 g de 2-(2'-imidazolinyl) 4-terbutyl 6-allyl phénol sous forme de cristaux de point de fusion 132-4°C.

Chlorhydrate

10 g de base sont dissous dans le méthanol et additionnés d'éther chlorhydrique jusqu'à pH acide. La solution est ensuite concentrée sous vide et reprise à l'éther. Les cristaux formés sont

essorés puis lavés à l'éther, à l'acétone et séchés. Après recristallisation dans un mélange acétonitrile-eau 97,5-2,5 on obtient 9 g de chlorhydrate de 2-(2'-imidazolinyl) 4-terbutyl 6-allyl phénol sous forme de cristaux de point de fusion 211-4°C.

EXEMPLE 46

2-hydroxy 3-nitro 5-terbutyl benzonitrile

formule VII X = $NO_2$ R = terbutyle

On porte au reflux durant 1 h un mélange de 26,5 g de 2-hydroxy 3-nitro 5-terbutyl benzaldéhyde, 60 ml d'acide formique 98 %, 8,5 g de formiate de sodium et 8,5 g de chlorhydrate d'hydroxylamine.

Le mélange réactionnel est ensuite refroidi, repris par un mélange d'eau et de glace et extrait à l'éther. La phase éthérée est lavée à l'eau, avec une solution aqueuse de bicarbonate puis encore à l'eau et enfin séchée sur sulfate de sodium. Après filtration et évaporation de l'éther on récupère 26 g d'un résidu qui cristallise dans l'éther isopropylique. Les cristaux sont essorés, lavés à l'éther isopropylique puis au pentane et séchés. On récupère ainsi 19,2 g de 2-hydroxy 3-nitro 5-terbutyl benzonitrile sous forme de cristaux de point de fusion 103-5°C.

EXEMPLE 47

2-hydroxy 3-amino 5-terbutyl benzonitrile

formule VII X = $NH_2$ R = terbutyle

On soumet à l'hydrogénation à pression ordinaire et à température ambiante en présence de Ni/Raney une solution de 9,2 g de 2-hydroxy 3-nitro 5-terbutyl benzonitrile obtenu à l'exemple 46 dans 250 ml d'éthanol.

Après absorption de la théorie d'hydrogène le mélange réactionnel est filtré pour séparer le catalyseur puis concentrer sous vide et le résidu repris dans le dichlorométhane est filtré sur gel de silice. On obtient ainsi 5 g de 2-hydroxy 3-amino 5-terbutyl benzonitrile sous forme de cristaux de point de fusion 132-6°C.

EXEMPLE 48

2-(2'-imidazolinyl) 4-terbutyl 6-aminophénol

formule I n = 0 R = terbutyle X = $NH_2$

On porte à 165°C en tube scellé durant 8 h un mélange de 5 g de 2-hydroxy 3-amino 5-terbutyl benzonitrile préparé à l'exemple 47 et 15 ml d'éthylène diamine.

Le mélange réactionnel est ensuite amené à sec sous vide et le résidu cristallise dans l'acétonitrile.

Les cristaux sont essorés et lavés avec de l'acétonitrile, et séchés. On récupère ainsi 3 g de 2-(2'-imidazolinyl) 4-terbutyl 6-aminophénol sous forme de cristaux de point de fusion 211-213°C.

EXEMPLE 49

Chlorhydrate de 2-(2'-imidazolinyl) 4-terbutyl 6-acétamido phénol

formule I n = 0 R = terbutyle X = $-NHCOCH_3$

A une solution de 3 g de 2-(2'-imidazolinyl) 4-terbutyl 6-aminophénol préparé à l'exemple 48 dans 20 ml d'acide acétique on ajoute goutte à goutte à température ambiante une solution de 1,3 ml d'anhydride acétique dans 10 ml d'acide acétique.

Après la fin de l'addition on agite 6 h à température ambiante puis on laisse au repos une nuit.

Le mélange réactionnel est ensuite additionné d'éther chlorhydrique puis évaporé à sec. Le résidu est ensuite repris à chaud par 100 ml d'isopropanol et les cristaux formés sont essorés. On récupère ainsi 2,3 g du chlorhydrate de 2-(2'-imidazolinyl) 4-terbutyl 6-acétamido phénol sous forme de cristaux de point de fusion 149-151°C.

EXEMPLE 50

2-(2'-imidazolinyl) 4-isobutyl 6-nitrophénol

formule I n = 0 R = isobutyle X = $NO_2$

Selon le mode opératoire de l'exemple 35 mais à partir de 16 g de 2-(2'-imidazolinyl) 4-isobutyl phénol préparé à l'exemple 9 du ler texte on obtient après recristallisation dans le méthanol 6,6 g de 2-(2'-imidazolinyl) 4-isobutyl 6-nitrophénol sous forme de cristaux de point de fusion 293°C décomposition.

EXEMPLE 51

2-hydroxy 3-thiométhyl 5-isobutyl benzaldéhyde.

formule VIII X = $SCH_3$ R = isobutyle

Selon la méthode de DUFF, en utilisant le mode opératoire de l'exemple 32, mais à partir de 118 g de 2-thiométhyl 4-isobutyl

phénol on récupère 34,6 g de 2-hydroxy 3-thiométhyl 5-isobutyl benzaldéhyde sous forme d'un liquide qu'on utilise brut pour l'étape suivante.

EXEMPLE 52

2-hydroxy 3-thiométhyl 5-isobutyl benzonitrile

formule VII X = $SCH_3$ R = isotubyle

Selon le mode opératoire de l'exemple 30 mais en utilisant 34,6 g de 2-hydroxy 3-thiométhyl 5-isobutyl benzaldéhyde préparé à l'exemple 51 on récupère 27,4 g de 2-hydroxy 3-thiométhyl 5-isobutyl benzonitrile sous forme de cristaux de point de fusion 74-7°C

EXEMPLE 53

2-(2'-imidazolinyl) 4-isobutyl 6-thiométhyl phénol

formule I n = 0 R = isobutyle X = $SCH_3$

Selon le mode opératoire de l'exemple 31 mais en utilisant 27,4 g de 2-hydroxy 3-thiométhyl 5-isobutyl benzonitrile préparé à l'exemple 52 et 62 ml d'éthylène diamine, on récupère 20,5 g de 2-(2'-imidazolinyl) 4-isobutyl 6-thiométhyl phénol sous forme de cristaux de point de fusion 195-7°C.

Chlorhydrate

10 g de base sont dissous dans le méthanol et additionnés d'éther chlorhydrique. Après évaporation de la solution le résidu est repris à l'éther et les cristaux formés sont essorés, lavés à l'éther et séchés. Par recristallisation dans l'acétonitrile on récupère 9,6 g de chlorhydrate de 2-(2'-imidazolinyl) 4-isobutyl 6-thiométhyl phénol sous forme de cristaux de point de fusion 113-116°C.

EXEMPLE 54

2-(2'-imidazolinyl) 4-isobutyl 6-méthyl sulfinyl phénol

formule I n = 0 R = isobutyle X = $SOCH_3$

Selon le mode opératoire de l'exemple 39 mais à partir de 10,5 g de 2-(2'-imidazolinyl) 4-isobutyl 6-thiométhyl phénol préparé à l'exemple 53, on obtient 5,2 g de 2-(2'-imidazolinyl) 4-isobutyl 6-méthyl sulfinyl phénol sous forme de cristaux de point de fusion 198-201°C.

maléate

Les 5,2 g de base sont dissous dans le minimum de méthanol et additionnés de 2,1 g d'acide maléique dissous dans le minimum de méthanol. La solution est évaporée et le résidu repris par un mélange acétone-éther cristallise. Les cristaux sont essorés, lavés à l'éther et séchés. On récupère ainsi 6,3 g de maléate de 2-(2'-imidazolinyl) 4-isobutyl 6-méthyl sulfinyl phénol sous forme de cristaux de point de fusion 112-116°C.

EXEMPLE 55

p-méthyl cyclopentyl o-chlorométhyl anisole

formule V R = 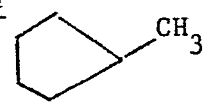

On porte à 60°C durant 14 h un mélange de 197 g de p-méthylcyclopentyl anisole, 31 g de formol (trioxy méthylène) et 346 ml d'acide chlorhydrique 36 %.

Le mélange réactionnel est ensuite refroidi et extrait à l'éther.

La phase éthérée est ensuite lavée à l'eau, à la soude 5 % puis encore à l'eau et séchée sur sulfate de sodium. L'éther est évaporé et le résidu est soumis à distillation sous vide. On récupère ainsi 153 g de p-méthylcyclopentyl o-chlorométhyl anisole Eb = 140-160°C/ 4 mm Hg.

EXEMPLE 56

2-méthoxy 5-méthylcyclopentyl benzaldéhyde

formule IV R = 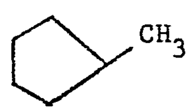

A une solution de méthylate de sodium préparée à partir de 14,8 g de sodium dans 640 ml de méthanol, on ajoute goutte à goutte 59 g de nitro-2 propane. On laisse le mélange sous agitation durant 30 minutes puis on additionne goutte à goutte, en maintenant la température inférieure à 45°C, 153 g de p-méthyl cyclopentyl o-chlorométhyl anisole préparé à l'exemple 55. Après la fin de l'addition le mélange est agité durant 3 h en maintenant la température entre 40 et 45°C.

Le mélange réactionnel est ensuite concentré sous vide, repris à l'eau et extrait à l'éther. La phase éthérée est soigneusement lavée à l'eau, à la soude 10 % et encore à l'eau puis séchée sur sulfate de sodium.

L'éther est évaporé après filtration et le résidu obtenu est soumis à distillation sous vide. On récupère ainsi 110,3 g de 2-méthoxy 5-méthylcyclopentyl benzaldéhyde sous forme d'un liquide Eb = 140-150°C/2 mm Hg.

EXEMPLE 57

2-méthoxy 5-méthylcyclopentyl benzonitrile

formule III R = 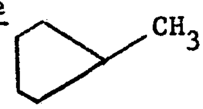

Selon le mode opératoire décrit à l'exemple 46 mais en utilisant 110,3 g de 2-méthoxy 5-méthylcyclopentyl benzaldéhyde préparé à l'exemple 56, 76 ml d'acide formique 98 %, 36,2 g de chlorhydrate d'hydroxylamine et 36,2 g de formiate de sodium, on obtient après distillation sous vide 71,6 g de 2-méthoxy 5-méthylcyclopentyl benzonitrile sous forme d'un liquide Eb = 158-168°C/1,5 mm Hg.

EXEMPLE 58

2-(2'-imidazolinyl) 4-méthyl cyclopentyl phénol

formule II n = 0 R = 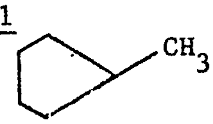

On porte à 165°C en tube scellé, 8 h, un mélange de 35,6 g de 2-méthoxy 5-méthylcyclopentyl benzonitrile préparé à l'exemple 57 et 36 ml d'éthylène diamine.

Le mélange réactionnel est ensuite refroidi et les cristaux formés sont essorés et soigneusement lavés à l'isopropanol puis à l'éther et séchés. On récupère ainsi 27,8 g de 2-(2'-imidazolinyl) 4-méthylcyclopentylphénol sous forme de cristaux de point de fusion 223-5°C.

EXEMPLE 59

Chlorhydrate de 2-(2'-imidazolinyl) 4-méthylcyclopentyl 6-iodophénol

formule I n = 0 R = 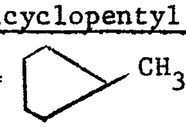 X = I

A une solution de 8,5 g de 2-(2'-imidazolinyl) 4-méthylcyclopentyl phénol préparé à l'exemple 58 dans 60 ml d'acide acétique glacial, on ajoute par petites fractions 6,2 g de chlorure d'iode sous bonne agitation. Après la fin de l'addition, l'agitation est continuée durant 5 h puis le mélange réactionnel est laissé au repos durant une nuit. Les cristaux formés sont

ensuite essorés, lavés avec un peu d'acide acétique, avec de l'acétonitrile puis à l'éther et séchés. On récupère ainsi 5,8 g de chlorhydrate de 2-(2'-imidazolinyl) 4-méthylcyclopentyl 6-iodophénol sous forme de cristaux de point de fusion 185-187°C.

EXEMPLE 60

Bromhydrate de 2-(2'-imidazolinyl) 4-méthylcyclopentyl 6-bromophénol

formule I n = 0 R = 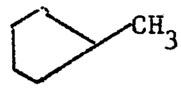 X = Br

A une solution de 8 g de 2-(2'-imidazolinyl) 4-méthylcyclopentyl phénol préparé à l'exemple 58 dans 60 ml d'acide acétique glacial, on ajoute goutte à goutte, sous bonne agitation, une solution de 1,8 ml de brome dans 10 ml d'acide acétique glacial. Après la fin de l'addition, on continue l'agitation. Au bout de 1 h, on constate que le produit commence à cristalliser. On continue l'agitation encore 4 h puis les cristaux formés sont essorés, lavés avec un peu d'acide acétique, d'acétonitrile puis à l'éther et séchés. Après recristallisation dans un mélange de 200 ml d'acétonitrile et 10 ml d'isopropanol, on récupère 7,4 g de bromhydrate de 2-(2'-imidazolinyl) 4-méthylcyclopentyl 6-bromophénol sous forme de cristaux de point de fusion 211-4°C.

EXEMPLE 61

2-hydroxy 5-méthylcyclopentyl benzaldéhyde

formule VIII X = H R = 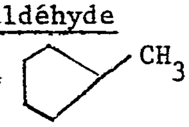

Selon la méthode de DUFF, en utilisant le mode opératoire de l'exemple 32 mais à partir de 100 g de p-méthylcyclopentyl phénol on récupère 31,1 g de 2-hydroxy 5-méthylcyclopentyl benzaldéhyde sous forme d'une huile qu'on utilise brute pour l'étape suivante.

EXEMPLE 62

2-hydroxy 3-chloro 5-méthylcyclopentyl benzaldéhyde

formule VIII X = Cl R = 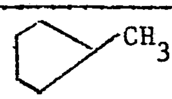

A une solution de 30,6 g de 2-hydroxy 5-méthylcyclopentyl benzaldéhyde préparé à l'exemple 61 dans 300 ml de chloroforme, on ajoute goutte à goutte sous agitation une solution de 15 g de chlore dans 600 ml de chloroforme. Après la fin de l'addition

l'agitation est maintenue durant 6 h puis la solution est laissée au repos une nuit. Elle est ensuite soigneusement lavée à l'eau puis séchée et concentrée sous vide. Le résidu est repris à l'isopropanol et les cristaux formés sont essorés et lavés avec un peu d'isopropanol puis au pentane et séchés. On récupère ainsi 15,2 g de 2-hydroxy 3-chloro 5-méthylcyclopentyl benzaldéhyde sous forme de cristaux de point de fusion 73-4°C.

EXEMPLE 63

2-hydroxy 3-chloro 5-méthylcyclopentyl benzonitrile

formule VII   X = Cl   R = 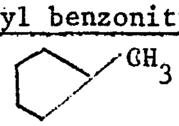

Selon le mode opératoire de l'exemple 30 mais en utilisant 15,2 g de 2-hydroxy 3-chloro 5-méthylcyclopentyl benzaldéhyde préparé à l'exemple 62 (oxime : F = 163-5°C) on récupère 13,4 g de 2-hydroxy 3-chloro 5-méthylcyclopentyl benzonitrile sous forme de cristaux de point de fusion 93-4°C.

EXEMPLE 64

2-(2'-imidazolinyl) 4-méthylcyclopentyl 6-chlorophénol

formule I   n = 0   R = 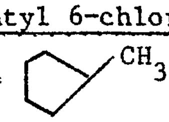   X = Cl

Selon le mode opératoire de l'exemple 31 mais en utilisant 13,4 g de 2-hydroxy 3-chloro 5-méthylcyclopentyl benzonitrile préparé à l'exemple 63 et 32 ml d'éthylène diamine on récupère 12,5 g de 2-(2'-imidazolinyl) 4-méthylcyclopentyl 6-chlorophénol sous forme de cristaux de point de fusion 288-294°C.

Chlorhydrate

Ces 12,5 g de base sont dissous dans le méthanol et additionnés d'éther chlorhydrique jusqu'à pH acide. La solution est concentrée sous vide et le résidu repris à l'éther. Les cristaux formés, sont essorés, lavés à l'éther, à l'acétonitrile puis encore à l'éther et séchés. On récupère ainsi 13,3 g du chlorhydrate de 2-(2'-imidazolinyl) 4-méthylcyclopentyl 6-chlorophénol sous forme de cristaux de point de fusion 228-232°C.

EXEMPLE 65

p-méthyl o-chlorométhyl anisole

formule V   R = $CH_3$

Selon le mode opératoire de l'exemple 55 mais en utilisant 70,3 g de p-méthyl anisole on récupère 79,7 g de p-méthyl o-chlorométhyl anisole sous forme de liquide Eb = 116-125°C/10 mm Hg.

EXEMPLE 66

2-méthoxy 5-méthyl benzaldéhyde

formule IV     R = $CH_3$

Selon le mode opératoire décrit à l'exemple 56 mais à partir de 79,7 g de p-méthyl o-chlorométhyl anisole préparé à l'exemple 65 et 42,9 g de nitro-2 propane sodé par une solution de méthylate de sodium préparée à partir de 10,8 g de sodium dans 465 ml de méthanol, on obtient après distillation sous vide 56,2 g de 2-méthoxy 5-méthyl benzaldéhyde Eb = 127-137°C/10 mm Hg.

EXEMPLE 67

2-méthoxy 5-méthyl benzonitrile

formule III     R = $CH_3$

Selon le mode opératoire décrit à l'exemple 46 mais en utilisant 56,2 g de 2-méthoxy 5-méthyl benzaldéhyde préparé à l'exemple 66, 56 ml d'acide formique 98 %, 26,7 g de chlorhydrate d'hydroxylamine et 26,7 g de formiate de sodium, on obtient après distillation sous vide 41,3 g de 2-méthoxy 5-méthyl benzonitrile sous forme d'un liquide Eb = 112-116°C/1,5 mm Hg.

EXEMPLE 68

2-(2'-imidazolinyl) 4-méthyl phénol

formule II     n = 0     R = $CH_3$

Selon le mode opératoire de l'exemple 58 mais en utilisant 41,3 g de 2-méthoxy 5-méthyl benzonitrile préparé à l'exemple 67 et 99 ml d'éthylène diamine, on obtient 37,1 g de 2-(2'-imidazolinyl) 4-méthyl phénol sous forme de cristaux de point de fusion 267-271°C.

EXEMPLE 69

Chlorhydrate de 2-(2'-imidazolinyl) 4-méthyl 6-iodophénol

formule I     n = 0     R = $CH_3$     X = I

Selon le mode opératoire de l'exemple 59 mais en utilisant 11 g de 2-(2'-imidazolinyl) 4-méthyl phénol préparé à l'exemple 68 et 10,3 g de chlorure d'iode, on obtient 6,5 g de chlorhydrate de 2-(2'-imidazolinyl) 4-méthyl 6-iodophénol sous

forme de cristaux de point de fusion 190-2°C.

EXEMPLE 70

Bromhydrate de 2-(2'-imidazolinyl) 4-méthyl 6-bromophénol

formule I n = 0 R = $CH_3$ X = Br

Selon le mode opératoire de l'exemple 60, mais en utilisant 11 g de 2-(2'-imidazolinyl) 4-méthyl phénol préparé à l'exemple 68 et 3,3 ml de brome, on obtient 17,5 g du bromhydrate de 2-(2'-imidazolinyl) 4-méthyl 6-bromo phénol sous forme de cristaux de point de fusion 249-250°C.

EXEMPLE 71

2-(2'-imidazolinyl) 4-méthyl 6-chlorophénol

formule I n = 0 R = $CH_3$ X = Cl

A une solution de 12,8 g de 2-(2'-imidazolinyl) 4-méthyl phénol préparé à l'exemple 68 dans 130 ml d'acide acétique glacial, on ajoute sous bonne agitation 11,5 ml de chlorure de sulfuryle goutte à goutte.

Après la fin de l'addition le mélange réactionnel est agité encore durant 6 h puis laissé au repos une nuit.

Les cristaux formés sont essorés, lavés avec un peu d'acide acétique, un peu d'isopropanol puis à l'éther et séchés. Les 9 g ainsi obtenus sont dissous dans l'eau et traités par une solution aqueuse d'ammoniaque. Les cristaux formés alors sont essorés, soigneusement lavés à l'eau et séchés. On récupère ainsi 6,4 g de 2-(2'-imidazolinyl) 4-méthyl 6-chlorophénol sous forme de cristaux de point de fusion 305-308°C.

Chlorhydrate

Les 6,4 g de base sont dissous dans le méthanol et additionnés d'éther chlorhydrique jusqu'à pH acide. La solution est concentrée sous vide et le résidu repris à l'éther cristallise. Les cristaux sont essorés, lavés à l'éther et séchés. Après recristallisation dans le mélange isopropanol-éthanol 75-25, on récupère 5 g de chlorhydrate de 2-(2'-imidazolinyl) 4-méthyl 6-chlorophénol sous forme de cristaux de point de fusion 263-270°C

EXEMPLE 72

p-éthyl o-chlorométhyl anisole

formule V R = $C_2H_5$

Selon le mode opératoire de l'exemple 55 mais en utilisant 143,1 g de p-éthylanisole on récupère 163,8 g de p-éthyl o-chlorométhylanisole sous forme de liquide Eb = 115-125°C/5 mm Hg.

EXEMPLE 73

2-méthoxy 5-éthyl benzaldéhyde

formule IV R = $C_2H_5$

Selon le mode opératoire de l'exemple 56 mais à partir de 163,8 g de p-éthyl o-chlorométhylanisole préparé à l'exemple 72 et 81,6 g de nitro-2 propane sodé par une solution de méthylate de sodium préparée à partir de 20,4 g de sodium dans 890 ml de méthanol, on obtient après distillation sous vide 116,3 g de 2-méthoxy 5-éthyl benzaldéhyde Eb = 123-131°C/5 mm Hg.

EXEMPLE 74

2-méthoxy 5-éthyl benzonitrile

formule III R = $C_2H_5$

Selon le mode opératoire décrit à l'exemple 46 mais en utilisant 95 g de 2-méthoxy 5-éthyl benzaldéhyde préparé à l'exemple 73, 86 ml d'acide formique 98%, 41,2 g de chlorhydrate d'hydroxylamine et 41,2 g de formiate de sodium, on obtient après distillation sous vide 67,5 g de 2-méthoxy 5-éthyl benzonitrile sous forme d'un liquide Eb = 115-130°C/2 mm Hg.

EXEMPLE 75

2-(2'-imidazolinyl) 4-éthyl phénol

formule II n = 0 R = $C_2H_5$

Selon le mode opératoire de l'exemple 58, mais en utilisant 67,5 g de 2-méthoxy 5-éthyl benzonitrile préparé à l'exemple 74 et 162 ml d'éthylène diamine, on obtient 59,8 g de 2-(2'-imidazolinyl) 4-éthyl phénol sous forme de cristaux de point de fusion 208-213°C.

EXEMPLE 76

Chlorhydrate de 2-(2'-imidazolinyl) 4-éthyl 6-iodophénol

formule I n = 0 R =$C_2H_5$ X = I

Selon le mode opératoire de l'exemple 59 mais en utilisant 11 g de 2-(2'-imidazolinyl) 4-éthyl phénol préparé à l'exemple 75 et 9,9 g de chlorure d'iode, on obtient 6,1 g de chlorhydrate de 2-(2'-imidazolinyl) 4-éthyl 6-iodophénol sous forme de cristaux de point de fusion 175-6°C.

EXEMPLE 77

Bromhydrate de 2-(2'-imidazolinyl) 4-éthyl 6-bromophénol

formule I    n = 0    R = $C_2H_5$    X = Br

Selon le mode opératoire de l'exemple 60 mais en utilisant 11 g de 2-(2'-imidazolinyl) 4-éthyl phénol préparé à l'exemple 75 et 3,1 ml de brome, on obtient 18 g du bromhydrate de 2-(2'-imidazolinyl) 4-éthyl 6-bromophénol sous forme de cristaux de point de fusion 241-3°C.

EXEMPLE 78

2-(2'-imidazolinyl) 4-éthyl 6-chlorophénol

formule I    n = 0    R = $C_2H_5$    X = Cl

Selon le mode opératoire de l'exemple 71 mais en utilisant 13,9 g de 2-(2'-imidazolinyl) 4-éthyl phénol préparé à l'exemple 75 et 11,5 ml de chlorure de sulfuryle on récupère 7,1 g de 2-(2'-imidazolinyl) 4-éthyl 6-chlorophénol sous forme de cristaux de point de fusion 284-7°C.

Chlorhydrate

Les 7,1 g de base sont dissous dans le méthanol et additionnés d'éther chlorhydrique jusqu'à pH acide. La solution est concentrée sous vide et le résidu repris à l'éther cristallise. Les cristaux sont essorés, lavés à l'éther et séchés. Après recristallisation dans un mélange acétonitrile-eau 94-6, on récupère 6,5 g du chlorhydrate de 2-(2'-imidazolinyl) 4-éthyl 6-chlorophénol sous forme de cristaux de point de fusion 243-6°C.

EXEMPLE 79

2-hydroxy 5-éthyl benzaldéhyde

formule VIII    X = H    R = $C_2H_5$

Selon la méthode de DUFF, en utilisant le mode opératoire de l'exemple 32 mais à partir de 200 g de p-éthyl phénol, on récupère 53,2 g de 2-hydroxy 5-éthyl benzaldéhyde sous forme d'une huile qu'on utilise brute pour l'étape suivante.

EXEMPLE 80

2-hydroxy 3-allyl 5-éthyl benzaldéhyde

formule VIII    X = allyle    R = $C_2H_5$

Selon le mode opératoire de l'exemple 43 mais en utilisant 15 g de 2-hydroxy 5-éthyl benzaldéhyde préparé à l'exemple 79 et

10,1 ml de bromure d'allyle, on obtient après le réarrangement de Claisen 12 g de 2-hydroxy 3-allyl 5-éthyl benzaldéhyde sous forme d'une huile qu'on utilise brute pour la suite des opérations.

EXEMPLE 81

2-hydroxy 3-allyl 5-éthyl benzonitrile

formule VII X = allyle R = $C_2H_5$

Selon le mode opératoire de l'exemple 30 mais en utilisant 15,2 g de 2-hydroxy 3-allyl 5-éthyl benzaldéhyde préparé à l'exemple 80, on récupère 10,1 g de 2-hydroxy 3-allyl 5-éthyl benzonitrile sous forme de cristaux de point de fusion 28-30°C.

EXEMPLE 82

2-(2'-imidazolinyl) 4-éthyl 6-allyl phénol

formule I n = 0 R = $C_2H_5$ X = allyle

Selon le mode opératoire de l'exemple 31 mais en utilisant 10,2 g de 2-hydroxy 3-allyl 5-éthyl benzonitrile préparé à l'exemple 81 et 25 ml d'éthylène diamine on récupère 5,6 g de 2-(2'-imidazolinyl) 4-éthyl 6-allyl phénol sous forme de cristaux de point de fusion 115-117°C.

Chlorhydrate

5,6 g de base sont dissous dans le méthanol et additionnés d'éther chlorhydrique jusqu'à pH acide. La solution est concentrée sous vide et le résidu repris à l'éther cristallise. Les cristaux sont essorés, lavés à l'éther, à l'acétonitrile, encore à l'éther et séchés. Après recristallisation dans un mélange acétonitrile-eau 97,5-2,5 on obtient 4,5 g de chlorhydrate de 2-(2'-imidazolinyl) 4-éthyl 6-allyl phénol sous forme de cristaux de point de fusion 207-211°C.

EXEMPLE 83

p-isopropyl o-chlorométhyl anisole

formule V R = isopropyle

Selon le mode opératoire de l'exemple 55 mais en utilisant 195,1 g de p-isopropyl anisole, on récupère 205,4 g de p-isopropyl o-chlorométhyl anisole sous forme de liquide Eb = 123-140°C/10 mm Hg.

EXEMPLE 84

2-méthoxy 5-isopropyl benzaldéhyde

formule IV R = isopropyle

Selon le mode opératoire de l'exemple 56 mais à partir de 205,4 g de p-isopropyl o-chlorométhyl anisole préparée à l'exemple 83 et 95 g de nitro-2 propane sodé par une solution de méthylate de sodium, préparée à partir de 23,8 g de sodium dans 1000 ml de méthanol, on obtient après distillation sous vide 159 g de 2-méthoxy 5-isopropyl benzaldéhyde Eb = 135-145°C/10 mm Hg.

EXEMPLE 85

2-méthoxy 5-isopropyl benzonitrile

formule III R = isopropyle

Selon le mode opératoire décrit à l'exemple 46 mais en utilisant 80 g de 2-méthoxy 5-isopropyl benzaldéhyde préparé à l'exemple 84, 67 ml d'acide formique 98 %, 32 g de chlorhydrate d'hydroxylamine et 32 g de formiate de sodium, on obtient après distillation sous vide 57,9 g de 2-méthoxy 5-isopropyl benzonitrile sous forme d'un liquide Eb = 115-130°C/1 mm Hg.

EXEMPLE 86

2-(2'-imidazolinyl) 4-isopropyl phénol

formule II n = 0 R = isopropyl

Selon le mode opératoire de l'exemple 58 mais en utilisant 40 g de 2-méthoxy 5-isopropyl benzonitrile préparé à l'exemple 85 et 96 ml d'éthylène diamine, on obtient 22 g de 2-(2'-imidazolinyl) 4-isopropyl phénol sous forme de cristaux de point de fusion 175-180°C.

EXEMPLE 87

Chlorhydrate de 2-(2'-imidazolinyl) 4-isopropyl 6-iodophénol

formule I n = 0 R = isopropyle X = I

Selon le mode opératoire de l'exemple 59 mais en utilisant 11 g de 2-(2'-imidazolinyl) 4-isopropyl phénol préparé à l'exemple 86 et 9,2 g de chlorure d'iode, on obtient 11,3 g de chlorhydrate de 2-(2'-imidazolinyl) 4-isopropyl 6-iodophénol sous forme de cristaux de point de fusion 173-175°C.

EXEMPLE 88

Bromhydrate de 2-(2'-imidazolinyl) 4-isopropyl 6-bromophénol

formule I n = 0 R = isopropyle X = Br

Selon le mode opératoire de l'exemple 60 mais en utilisant 11 g de 2-(2'-imidazolinyl) 4-isopropyl phénol préparé à l'exemple 86 et 2,9 ml de brome, on obtient 16,3 g de bromhydrate de 2-(2'-imidazolinyl) 4-isopropyl 6-bromophénol sous forme de cristaux de point de fusion 232-233°C.

EXEMPLE 89

2-(2'-imidazolinyl) 4-isopropyl 6-chlorophénol

formule I n = 0 R = isopropyle X = Cl

Selon le mode opératoire de l'exemple 71 mais en utilisant 11,7 g de 2-(2'-imidazolinyl) 4-isopropyl phénol préparé à l'exemple 86 et 9,1 ml de chlorure de sulfuryle, on récupère 7,7 g de 2-(2'-imidazolinyl) 4-isopropyl 6-chlorophénol sous forme de cristaux de point de fusion 242-5°C.

Chlorhydrate

Les 7,7 g de base sont dissous dans le méthanol et additionnés d'éther chlorhydrique jusqu'à pH acide. La solution est concentrée sous vide et le résidu repris à l'éther cristallise. Les cristaux sont essorés, lavés à l'éther et séchés. Après recristallisation dans un mélange acétonitrile-eau 97,5-2,5 on récupère 6,4 g de chlorhydrate de 2-(2'-imidazolinyl) 4-isopropyl 6-chlorophénol sous forme de cristaux de point de fusion 225-227°C.

EXEMPLE 90

2-(2'-imidazolinyl) 6-chlorophénol

formule I n = 0 R = H X = Cl

Selon le mode opératoire de l'exemple 31 mais à partir de 10 g de 2-hydroxy 3-chloro benzonitrile et 25 ml d'éthylène diamine, on obtient 10,8 g de 2-(2'-imidazolinyl) 6-chlorophénol sous forme de cristaux de point de fusion 355-360°C.

Chlorhydrate

Les 10,8 g de base sont dissous dans le méthanol et additionnés d'éther chlorhydrique jusqu'à pH acide. La solution est concentrée sous vide, et le résidu repris à l'éther cristallise. Les cristaux sont essorés, lavés à l'éther et séchés. On obtient ainsi 12,5 g de chlorhydrate de 2-(2'-imidazolinyl) 6-chlorophénol sous forme de cristaux de point de fusion 249-253°C.

EXEMPLE 91

2-(2'-imidazolinyl) 4,6 dichlorophénol

formule I n = 0 R = Cl X = Cl

Selon le mode opératoire de l'exemple 31 mais à partir de 16 g de 2-hydroxy 3,5-dichloro benzonitrile et 40 ml d'éthylène diamine on obtient 13 g de 2-(2'-imidazolinyl) 4,6 dichlorophénol sous forme de cristaux de point de fusion > 360°C.

Chlorhydrate

Les 13 g de base sont dissous dans le méthanol et additionnés d'éther chlorhydrique jusqu'à pH acide. La solution est concentrée sous vide et le résidu repris à l'éther cristallise. Les cristaux sont essorés, lavés à l'acétone puis à l'éther et séchés. On récupère ainsi 14,6 g de chlorhydrate de 2-(2'-imidazolinyl) 4,6-dichlorophénol sous forme de cristaux de point de fusion 249-252°C.

EXEMPLE 92

2-(2'-imidazolinyl) 6-méthoxy phénol

formule I n = 0 R = H X = $OCH_3$

Selon le mode opératoire de l'exemple 31, mais à partir de 46,5 g de 2-hydroxy 3-méthoxy benzonitrile et 100 ml d'éthylène diamine on obtient 48 g de 2-(2'-imidazolinyl) 6-méthoxy phénol sous forme de cristaux de point de fusion 303-308°C.

Chlorhydrate

15 g de base sont dissous dans le minimum de méthanol et additionnés d'éther chlorhydrique jusqu'à pH acide. On additionne à la solution un peu d'éther et on laisse cristalliser. Les cristaux formés sont essorés, lavés à l'éther et séchés. On récupère ainsi 14 g de chlorhydrate de 2-(2'-imidazolinyl) 6-méthoxy phénol sous forme de cristaux de point de fusion 223-226°C.

EXEMPLE 93

Bromhydrate de 2-(2'-imidazolinyl) 4-bromo 6-méthoxy phénol

formule I n = 0 R = Br X = $OCH_3$

Selon le mode opératoire de l'exemple 60 mais en utilisant 10 g de 2-(2'-imidazolinyl) 6-méthoxy phénol préparé à l'exemple 92 et 2,7 ml de brome, on obtient 16 g de bromhydrate de 2-(2'-imidazolinyl) 4-bromo 6-méthoxy phénol sous forme de cristaux de point de fusion 225-228°C.

EXEMPLE 94

Chlorhydrate de 2-(2'-imidazolinyl) 4-iodo 6-méthoxyphénol

formule I n = 0 R = I X = $OCH_3$

Selon le mode opératoire de l'exemple 59, mais en utilisant 10 g de 2-(2'-imidazolinyl) 6-méthoxy phénol préparé à l'exemple 92 et 8,5 g de chlorure d'iode, on obtient 8,4 g de chlorhydrate de 2-(2'-imidazolinyl) 4-iodo 6-méthoxy phénol sous forme de cristaux de point de fusion 205-8°C.

EXEMPLE 95

2-(2'-imidazolinyl) 4-méthoxy phénol

formule II n = 0 R = $OCH_3$

Selon le mode opératoire de l'exemple 31 mais à partir de 41,5 g de 2-hydroxy 5-méthoxy benzonitrile et 100 ml d'éthylène diamine, on obtient 27 g de 2-(2'-imidazolinyl) 4-méthoxy phénol sous forme de cristaux de point de fusion 230°C.

Chlorhydrate

10 g de base sont dissous dans le minimum de méthanol et additionnés d'éther chlorhydrique jusqu'à pH acide. On additionne à la solution un peu d'éther et on laisse cristalliser. Les cristaux formés sont essorés, lavés à l'éther et séchés. On récupère ainsi 7,7 g de chlorhydrate de 2-(2'-imidazolinyl) 4-méthoxy phénol sous forme de cristaux de point de fusion 279-280°C.

ACTIVITE ANTI-HYPERTENSIVE

1- Méthode

Des rats mâles âgés de 13 à 21 semaines présentant une hypertension spontanée génétiquement transmissible (souche OKAMOTO provenance IFFA-CREDO) sont placés dans une enceinte thermostatée à 38°C. Les rats sont laissés en situation libre. La pression artérielle est mesurée par un sphygmomanomètre Narco Biosystem. Un manchon est placé à la base de la queue et un détecteur enregistre le pouls en aval du manchon. Celui-ci est gonflé jusqu'à suppression du pouls, il est ensuite progressivement dégonflé. La pression mesurée à la reprise des battements cardiaques est la pression artérielle systolique. On calcule la fréquence cardiaque à partir de l'enregistrement du pouls artériel. Les produits à étudier

sont mis en suspension et administrés par voie orale sous un volume de 1 ml pour 100 g de poids corporel. Les mesures sont répétées à intervalles réguliers.

2- Résultats

Les valeurs moyennes avant traitement pour les différents lots de rats sont pour la pression artérielle systolique de 200 à 220 mm Hg et pour la fréquence cardiaque de 315 à 350 battements par minute.

Le Tableau II ci-après donne la moyenne des variations de la pression systolique (mm Hg), de la fréquence cardiaque (battements/minute) et les écarts à la moyenne obtenus 1 h, 3 h et 5 h après administration orale.

3- Conclusion

Les composés décrits possèdent une activité hypertensive remarquable car ils ne provoquent que peu ou pas de tachycardie réactionnelle. Ils sont peu toxiques : les doses létales 50 chez le rat par voie intrapéritonéale sont comprises entre 128 et 500 mg/kg ou supérieures à 500 mg/kg.

En thérapeutique humaine, les composés de formule (I) et leurs sels d'addition non toxiques peuvent être administrés notamment par voie orale ou injectable. Par voie orale, on préconise des gelules ou comprimés renfermant de 10 à 200 mg d'ingrédient actif et par voie injectable, des ampoules renfermant de 2 à 50 mg d'ingrédient actif.

Les composés de formule (I) permettent le traitement de l'hypertension, notamment sous forme de gélules ou sous une forme injectable.

TABLEAU I

5093-04

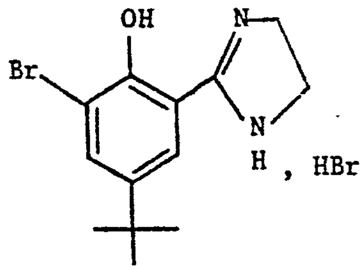

, HBr exemple 11

5093-05

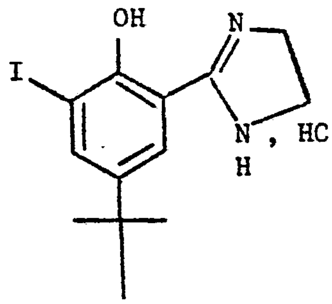

, HCl exemple 12

5093-06

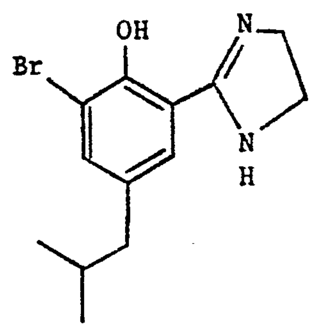

, HBr exemple 13

5093-07

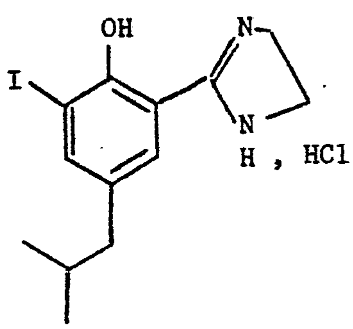

, HCl, $H_2O$ exemple 14

TABLEAU I (suite 1)

5093-08

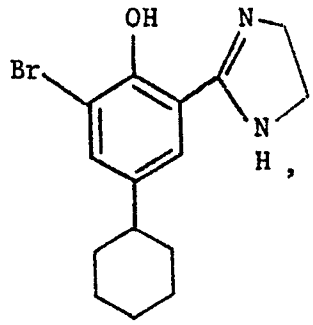

, H Br exemple 15

5093-09

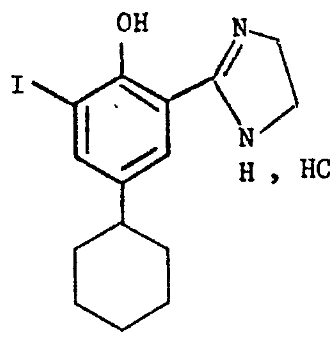

, HCl exemple 16

5093-11

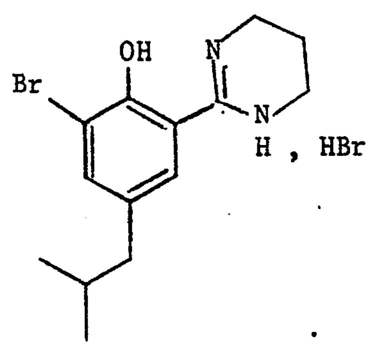

, HBr exemple 18

5093-39

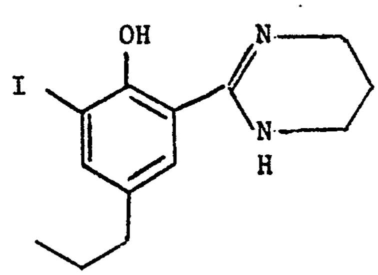

, HCl exemple 19

TABLEAU I (suite 2)

5093-13 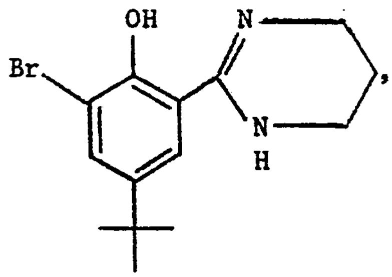 , HBr exemple 21

5093-14 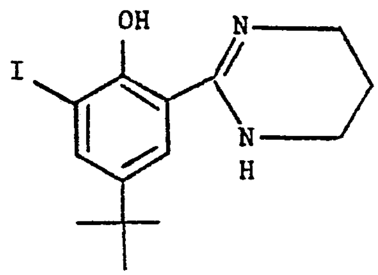 , HCl exemple 22

5093-19 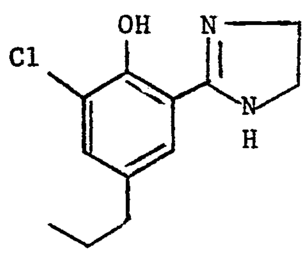 , HCl exemple 29

5093-34 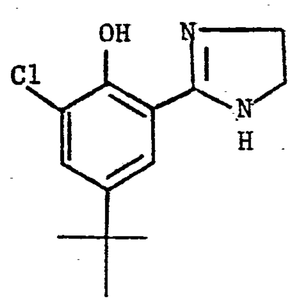 , HCl exemple 31

TABLEAU I (suite 3)

5093-55 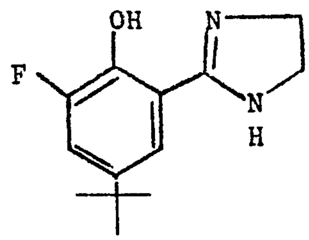 , HCl exemple 34

5093-60 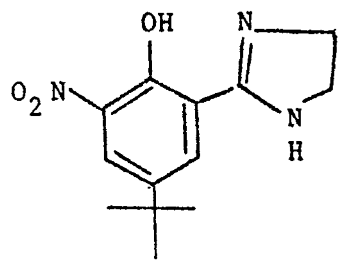 exemple 35

5093-64 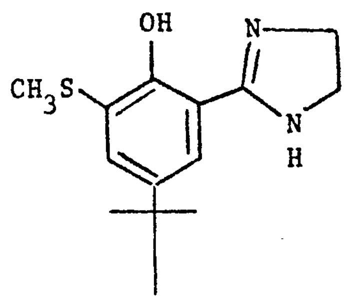 , HCl exemple 38

5093-71 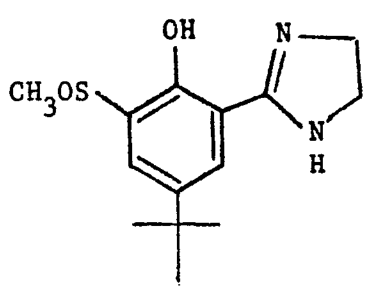 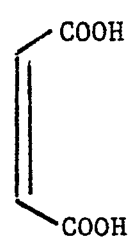 exemple 39

TABLEAU I (suite 4)

5093-70 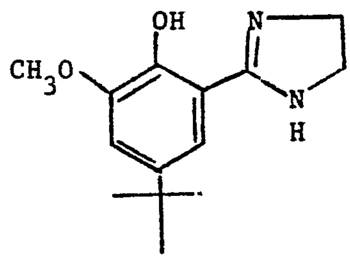 exemple 42

5093-73 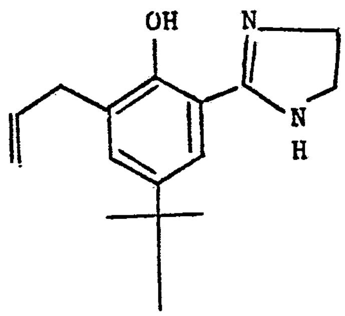 , HCl exemple 45

5093-67 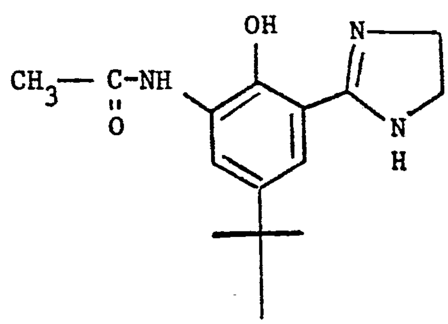 , HCl exemple 49

5093-49 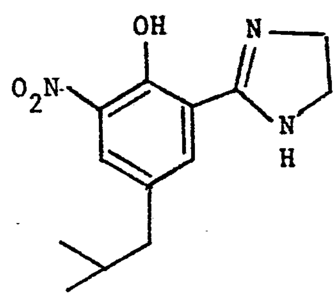 exemple 50

TABLEAU I (suite 5)

5093-72 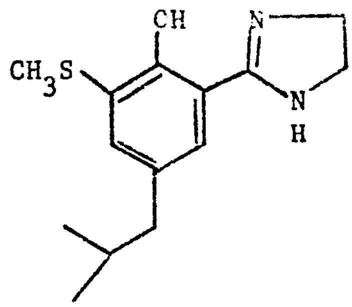 , HCl exemple 53

5093-74 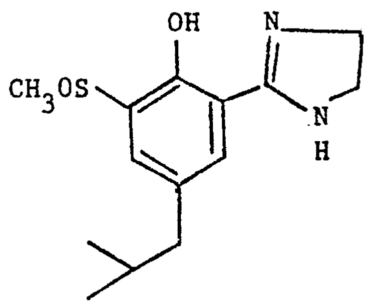 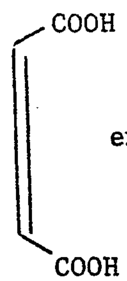 exemple 54

5093-32 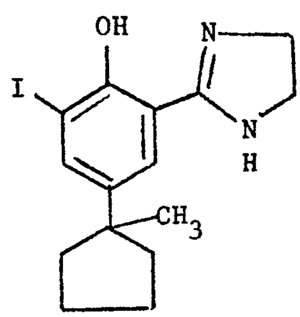 , HCl exemple 59

5093-35 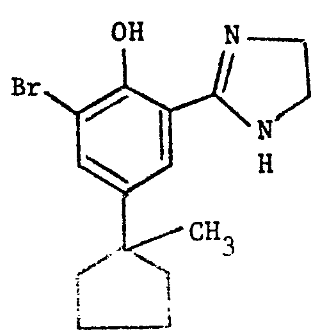 , HBr exemple 60

TABLEAU I (suite 6)

5093-63 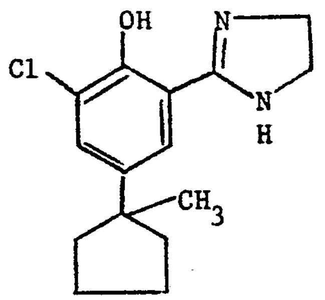 , HCl exemple 64

5093-59 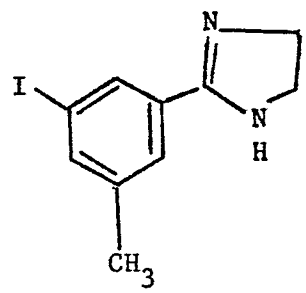 , HCl exemple 69

5093-58 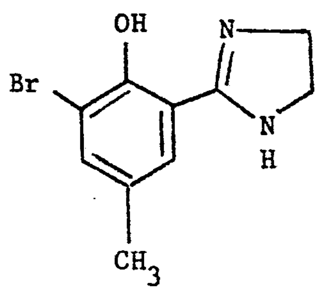 , HBr exemple 70

5093-65 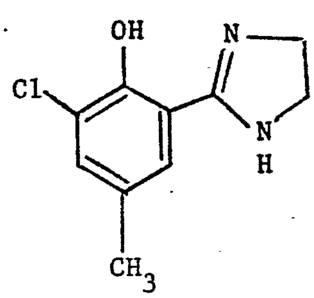 , HCl exemple 71

TABLEAU I (suite 7)

5093-62 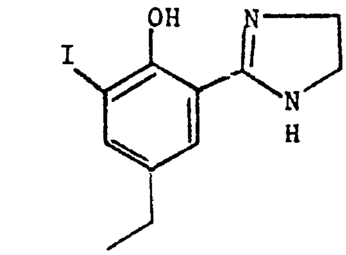 ,HCl exemple 76

5093-61 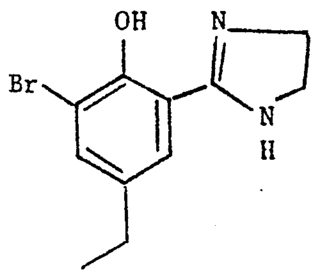 ,HBr exemple 77

5093-66 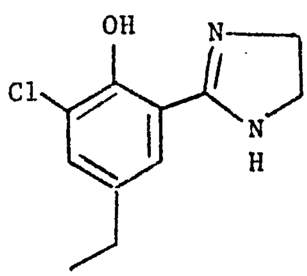 ,HCl exemple 78

5093-69 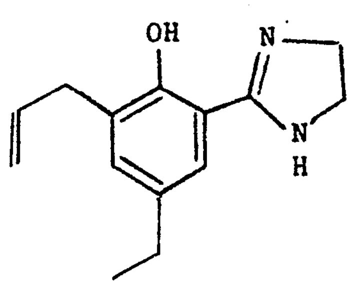 ,HCl exemple 82

TABLEAU I (suite 8)

5093-57 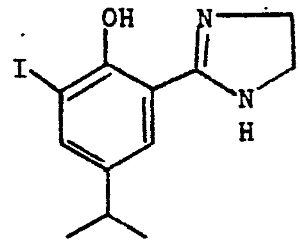 , HCl exemple 87

5093-56 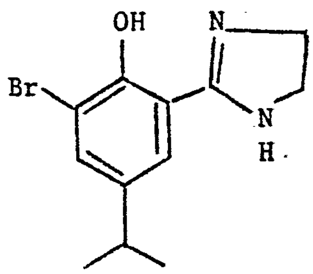 , HBr exemple 88

5093-68 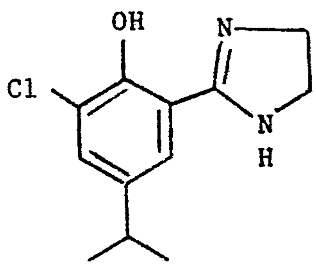 , HCl exemple 89

5116-04 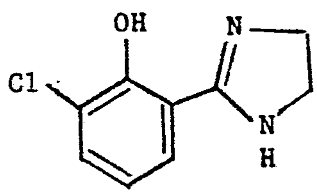 , HCl exemple 90

TABLEAU I (suite 9)

5116-12 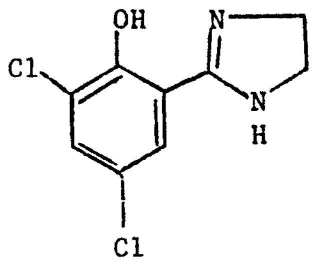 , HCl exemple 91

5116-08 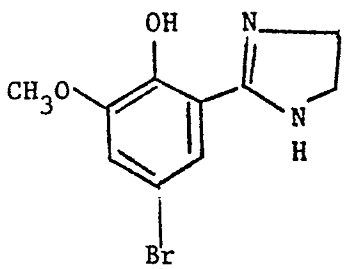 , HBr exemple 93

5116-09 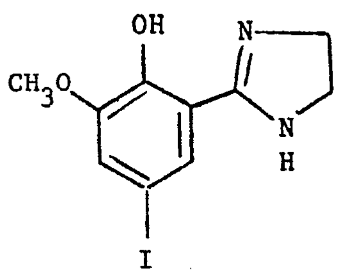 , HCl exemple 94

5116-06 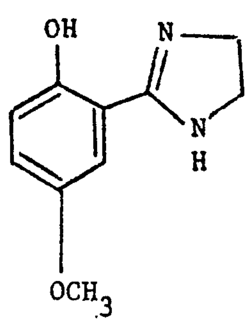 , HCl exemple 95

TABLEAU II

| Exemples | Nombre de rats/dose | Doses en mg/kg VO | Pression systolique Δ (mmHg) | | | Fréquence cardiaque Δ (battements/min) | | |
|---|---|---|---|---|---|---|---|---|
| | | | T + 1 heure | T + 3 heures | T + 5 heures | T + 1 heure | T + 3 heures | T + 5 heures |
| | | | m (sm) | m (sm) | m (sm) | m (sm) | m (sm) | m ( sm) |
| exemple 11 | 6 | 16 | -69 (13.6) | -75 (20.0) | -48 (11.8) | +37 (20.3) | -17 (8.0) | -10 (15.03) |
| | 6 | 32 | -108 (9.1) | -89 (10.9) | -80 (7.6) | +67 (11.2) | +33 (6.7) | -7 (6.7) |
| | 6 | 64 | -111 (12.1) | -108 (12.0) | -95 (12.0) | +54 (9.5) | +60 (16.3) | +51 (18.9) |
| | 5 | 128 | -133 (6.0) | -147 (9.3) | -145 (6.9) | +64 (25.6) | +20 (17.9) | +36 (27.9) |
| exemple 12 | 6 | 8 | -41 (6.2) | -32 (8.7) | -26 (9.3) | -3 (14.9) | -17 (12.0) | -30 (11.2) |
| | 6 | 16 | -34 (10.0) | -28 (13.7) | -26 (6.5) | -7 (12.3) | -7 (22.9) | -33 (17.7) |
| | 6 | 32 | -78 (14.0) | -73 (12.0) | -70 (4.7) | +13 (13.3) | -17 (12.0) | -10 (16.1) |
| | 6 | 64 | -107 (16.8) | -103 (14.6) | -87 (12.7) | +63 (15.8) | +37 (15.8) | +17 (9.5) |
| exemple 13 | 6 | 128 | -69 (11.8) | -78 (14.4) | -84 (11.9) | +3 (15.9) | +3 (22.8) | -17 (17.5) |
| exemple 14 | 6 | 128 | -52 (18.9) | -73 (16.1) | -58 (14.2) | +30 (11.3) | +20 (7.3) | +7 (11.2) |
| exemple 15 | 5 | 128 | -7 (6.8) | -36 (5.8) | -20 (2.0) | -13 (16.9) | -28 (5.8) | -33 (17.0) |
| exemple 16 | 6 | 128 | -11 (5.9) | -29 (7.4) | -18 (11.2) | +10 (13.4) | -23 (6.2) | -17 (12.3) |
| exemple 18 | 7 | 128 | -47 (9.3) | -53 (10.3) | -44 (8.8) | +16 (7.5) | +20 (10.7) | +27 (12.9) |

TABLEAU II (Suite 1)

| Exemples | Nombre de rats/dose | Doses en mg/kg$^{-1}$ VO | Pression artérielle systolique △ (mmHg) | | | Fréquence cardiaque Δ (battements/min) | | |
|---|---|---|---|---|---|---|---|---|
| | | | T + 1 heure | T + 3 heures | T + 5 heures | T + 1 heure | T + 3 heures | T + 5 heures |
| | | | m (sm) | m (sm) | m (sm) | m (sm) | m (sm) | m (sm) |
| Exemple 21 | 6 | 128 | -43 ± 13 | - 72 ± 9 | - 63 ± 10 | + 32 ± 15 | + 73 ± 14 | + 47 ± 17 |
| Exemple 22 | 6 | 128 | -58 ± 19 | - 73 ± 8 | - 77 ± 8 | + 53 ± 20 | + 60 ± 18 | + 58 ± 13 |
| Exemple 29 | 5 | 128 | -46 ± 7 | - 68 ± 15 | - 38 ± 12 | + 4 ± 13 | - 12 ± 15 | - 16 ± 11 |
| Exemple 31 | 5 | 64 | -98 ± 9 | -106 ± 12 | - 82 ± 15 | + 68 ± 17 | + 50 ± 10 | + 26 ± 18 |
| Exemple 34 | 6 | 32 | -24 ± 4 | - 19 ± 4 | - 19 ± 6 | - 25 ± 6 | - 2 ± 8 | - 2 ± 3 |
| Exemple 35 | 6 | 128 | -50 ± 1 | - 60 ± 4 | - 58 ± 6 | + 64 ± 17 | +103 ± 16 | + 94 ± 18 |
| Exemple 38 | 6 | 64 | -99 ± 7 | -105 ± 5 | - 80 ± 11 | + 98 ± 12 | + 68 ± 8 | + 35 ± 14 |

TABLEAU II (Suite 2)

| Exemples | Nombre de rats/dose | Doses en mg/kg$^{-1}$ VO | Pression artérielle systolique Δ (mmHg) | | | Fréquence cardiaque Δ (battements/min) | | |
|---|---|---|---|---|---|---|---|---|
| | | | T + 1 heure | T + 3 heures | T + 5 heures | T + 1 heure | T + 3 heures | T + 5 heures |
| | | | m (sm) | m (sm) | m (sm) | m (sm) | m (sm) | m (sm) |
| Exemple 49 | 6 | 128 | - 33 ± 10 | - 30 ± 10 | - 55 ± 6 | + 12 ± 19 | - 13 ± 12 | - 19 ± 16 |
| Exemple 59 | 6 | 128 | - 70 ± 11 | - 87 ± 16 | - 83 ± 13 | + 3 ± 23 | + 3 ± 22 | - 10 ± 18 |
| Exemple 60 | 6 | 128 | - 63 ± 19 | - 64 ± 12 | - 46 ± 10 | + 16 ± 12 | + 22 ± 14 | + 4 ± 14 |
| Exemple 64 | 6 | 64 | - 55 ± 8 | - 51 ± 10 | - 54 ± 10 | + 26 ± 16 | - 12 ± 12 | - 26 ± 13 |
| Exemple 70 | 6 | 32 | - 36 ± 9 | - 38 ± 16 | - 27 ± 6 | + 7 ± 16 | - 17 ± 15 | - 11 ± 13 |
| Exemple 71 | 6 | 128 | - 61 ± 6 | - 63 ± 11 | - 52 ± 9 | - 21 ± 33 | + 7 ± 15 | - 4 ± 20 |
| Exemple 77 | 6 | 64 | - 73 ± 12 | - 81 ± 9 | - 74 ± 7 | + 19 ± 21 | + 8 ± 19 | + 6 ± 16 |

TABLEAU II (suite 3)

| Exemples | Nombre de rats/dose | Doses en mg/kg$^{-1}$ VO | Pression artérielle systolique Δ (mmHg) | | | Fréquence cardiaque Δ(battements/min) | | |
|---|---|---|---|---|---|---|---|---|
| | | | T + 1 heure | T + 3 heures | T + 5 heures | T + 1 heure | T + 3 heures | T + 5 heures |
| | | | m (sm) | m (sm) | m (sm) | m (sm) | m (sm) | m (sm) |
| Exemple 78 | 6 | 128 | - 57 ± 12 | - 53 ± 21 | - 73±13 | + 26 ± 20 | + 67 ± 20 | + 10 ± 17 |
| Exemple 87 | 6 | 16 | 44±10 | - 27 ± 11 | - 24 ± 10 | + 2 ± 7 | - 10 ± 11 | - 32 ± 16 |
| Exemple 88 | 6 | 32 | - 69±12 | - 57 ± 9 | - 39 ± 9 | + 42 ± 17 | - 16 ± 21 | + 6 ± 16 |
| Exemple 90 | 6 | 32 | - 64 ± 10 | - 67 ± 9 | - 58 ± 7 | +118 ± 25 | + 97 ± 27 | + 76 ± 13 |
| Exemple 93 | 6 | 128 | - 27 ± 7 | - 21 ± 9 | - 19 ± 11 | - 4 ± 5 | - 6 ± 13 | - 11 ± 11 |
| Exemple 95 | 6 | 128 | - 25 ± 7 | - 15 ± 11 | - 30 ± 6 | + 34 ± 27 | + 30 ± 30 | + 20 ± 24 |

## REVENDICATIONS

1. Nouveaux composés caractérisés en ce qu'ils répondent à la formule :

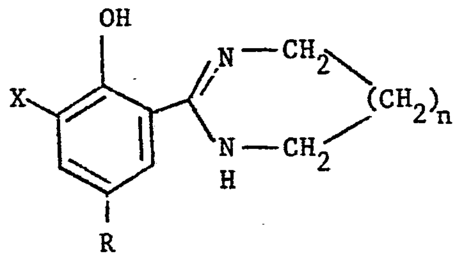

(I)

dans laquelle :

X représente un atome d'halogène, un groupement nitro, méthoxy, thiométhyl, $SOCH_3$, $SO_2CH_3$, acétamido ou allyle.

R représente un atome d'hydrogène, un radical alkyle notamment le radical terbutyle ou isobutyle, un radical cycloalkyle notamment le radical cyclohexyle, un radical alkylcycloalkyle notamment le radical méthylcyclopentyl, un radical alcoxy, un radical thioalkyle ou un atome d'halogène.

n est un nombre entier égal à 0, 1 ou 2,

et leurs sels d'addition d'acides.

2. Nouveaux composés selon la revendication 1, caractérisés en ce que R est choisi parmi : t-butyle, iso-butyle, cyclohexyle, méthylcyclopentyle, méthyle, éthyle, isopropyle, H, Cl, Br, I et $OCH_3$

3. Nouveaux composés selon la revendication 1 ou 2, caractérisés en ce que X est choisi parmi : Br, I, Cl, F, $NO_2$, $SCH_3$, $SOCH_3$, $OCH_3$, allyle, $NH_2$, et $-NHCOCH_3$.

4. Procédé de préparation des composés selon l'une quelconque des revendications 1 à 3, caractérisé en ce que l'on opère :

1 - soit par action d'un halogène ou d'un chlorure d'halogène sur un composé de formule (II) dans un solvant tel que par exemple l'acide acétique, à température ambiante, soit par action d'un N-halogénosuccinimide sur le compose de formule (II) dans un solvant tel que le diméthyl formamide, à température ambiante, soit par action du chlorure de sulfuryle dans un solvant tel que par exemple l'acide acétique, à température ambiante, soit par nitration à

l'aide de l'acide nitrique dans l'acide acétique, à température ambiante

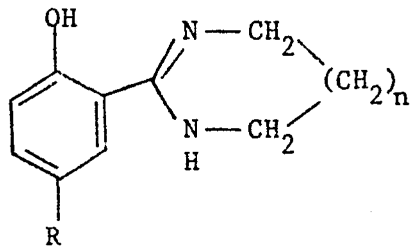

(II)

2 - soit par action d'une diamine de formule :

$$H_2N - CH_2 - (CH_2)_n - CH_2 - NH_2$$

où n est défini comme ci-dessus, à une température comprise entre 160°C et 250°C en tube scellé si nécessaire sur un phénol ortho nitrile de formule (VII) :

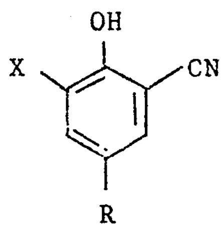

(VII)

R, dans les formules (II) et (VII), étant défini comme dans l'une quelconque des revendications 1 à 3,

5. Nouveaux médicaments utiles notamment comme anti-hypertenseurs, caractérisés en ce qu'ils consistent en les nouveaux composés selon l'une quelconque des revendications 1 à 3.

6. Compositions pharmaceutiques contenant au moins un médicament selon la revendication 5, en association avec un excipient pharmaceutiquement acceptable.

7. Nouveaux composés caractérisés en ce qu'ils consistent en les composés de formule :

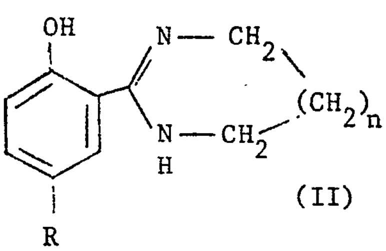

(II) ou

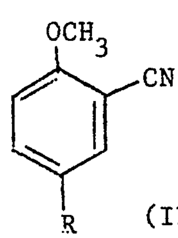

(III) ou

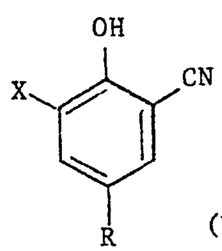

(VII)